**Europäisches Patentamt**

(19) **European Patent Office**

**Office européen des brevets**

(11) Veröffentlichungsnummer: **0 073 322 B1**

(12) **EUROPÄISCHE PATENTSCHRIFT**

(45) Veröffentlichungstag der Patentschrift:
05.02.86

(21) Anmeldenummer: 82106206.4

(22) Anmeldetag: 10.07.82

(51) Int. Cl.⁴: **G 01 M 3/18, G 01 M 3/00, G 01 N 27/12, F 17 D 5/06**

(54) Einrichtung zur Überwachung der Dichtheit von Dampfrohren.

(30) Priorität: 10.07.81 DE 3127244
10.07.82 DE 3225921

(43) Veröffentlichungstag der Anmeldung:
09.03.83 Patentblatt 83/10

(45) Bekanntmachung des Hinweises auf die Patenterteilung:
05.02.86 Patentblatt 86/6

(84) Benannte Vertragsstaaten:
AT BE CH DE FR GB IT LI LU NL SE

(56) Entgegenhaltungen:
EP - A - 0 045 888
EP - A - 0 053 382
DE - A - 3 018 781
DE - U - 8 011 887
US - A - 3 413 840

Kunststoff-Taschenbuch, Carl Hanser Verlag München Wien, 1979, S. 490 und S. 615
Patent Abstracts of Japan, Band 4, Nr. 18, 13. Februar 1980, Seite 47E171

(73) Patentinhaber: Barlian, Reinhold, Dieselstrasse 6,
D-6990 Bad Mergentheim (DE)

(72) Erfinder: Barlian, Reinhold, Dieselstrasse 6, D-6990 Bad Mergentheim (DE)
Erfinder: Fischle, Martin, Schönbornstrasse 21,
D-6990 Bad Mergentheim (DE)
Erfinder: Kastens, Friedhelm, Südstrasse 32,
D-7109 Krautheim 1 (DE)

LIBER, STOCKHOLM 1986

**Beschreibung**

Die Erfindung betrifft eine Überwachungseinrichtung mit den Merkmalen des Oberbegriffs des Anspruchs 1.

Zur Überwachung von Dampfrohren od. dgl. in Kraft,erken, insbesondere Kernkraftwerken, ist es aus der EP-A-0045888 veröffentlicht um 17.02.82. bekannt, sowohl Temperaturänderungen als auch Feuchtigkeitsänderungen zu überwachen, um beim Überschreiten eines Schwellwertes im Falle einer etwaigen Leckage an Systemteilen umgehend entsprechende Maßnahmen zur Schadensbehebung und Unfallverhütung einleiten zu können. Dazu ist es bekannt, etwaige Temperaturänderungen über einen Widerstandsdraht zu erfassen und Feuchtigkeitsschwankungen über einen hygroskopischen Isolator aufzunehmen, wobei durch einen unterschiedlichen Feuchtigkeitsgehalt entsprechende Widerstandsund/oder Kapazitätsänderungen erfaßt werden können. Diese an sich zuverlässige sowie funktionstüchtige Meß- bzw. Überwachungsmethode der beiden Faktoren Temperatur und Feuchtigkeit ist insofern nicht ganz zufriedenstellend, da die Herstellung und Anordnung des Meßsystems verhältnismäßig aufwendig ist. Dies insbesondere deshalb, weil für die Temperaturerfassung und für die Feuchtigkeitserfassung praktisch verschiedene und im wesentlichen getrennt voneinander angeordnete Erfassungssysteme angewandt werden. Das heißt, daß ein verhältnismäßig großer mechanischer Aufwand bei der Herstellung zu bewältigen ist, da eine Reihe verschiedener Bauteile zueinander in Verbindung gebracht werden müssen, was sich zudem auch nicht unwesentlich nachteilig auf die Herstellungskosten auswirken kann, da die Einzelbauteile teuer und die Montagearbeiten zeitaufwendig sind. Weiterhin ist ein verhältnismäßig großer Platzbedarf für die Meßanordnung erforderlich. Außerdem ist die Lebensdauer bzw. Funktionstüchtigkeit eingeschränkt, da aufgrund der vielfältigen Bauteile eine entsprechend große Gefahr für ein Versagen bzw. einen Ausfall eines Teils gegeben ist. Darüber hinaus beinhaltet das bisherige Meßsystem eine Störempfindlichkeit, die eine genaue Überwachung beeinträchtigen kann. Aus Patent Abstracts of Japan, Bd.4, Nr.18, l3.Februar 1980, S.47 E 171, ist ein Meßkabel bekannt, das zur Steuerung von Klimageräten vorgesehen ist. Mit dem Meßkabel sollen keine Leckagen, sondern der in der Umgebungsluft vorherrschende Temperatur- und Feuchtegehalt erfaßt werden, um die Klimaanlage entsprechend steuern zu können. Für diesen Zweck weist das Meßkabel zwei Widerstandsdrähte auf, die in entgegengesetzten Richtungen zueinander gewickelt sind und zwischen denen eine feuchtigkeitsaufnehmende Schicht, die aus Nylon oder ähnlichem bestehen kann, sich befindet. Außen weist das Meßkabel eine luftdurchlässige Umhüllung auf. Bei diesem nicht gattungsgemäßen Meßkabel ist innen kein elektrischer Leiterdraht vorgesehen. Der Kernstrang besteht vielmehr aus einem elektrischen Isolationsmaterial, insbesondere Tetoron. Tetoron ist der Handelsname eines Polyterephthalates, das zur Gruppe der Polyester gehört (Kunststoff-Taschenbuch, 21.Ausgabe, Karl Hanser Verlag München Wien 1979).

Demgemäß besteht die Aufgabe der Erfindung darin, eine Überwachungseinrichtung der eingangs beschriebenen Art so zu verbessern, daß eine wesentliche Einsparung verschiedener Bauteile, eine Platzersparnis sowie eine höhere Funktionstüchtigkeit erreicht werden kann.

Diese Aufgabe wird bei einer Einrichtung des eingangs genannten hat erfindrfindungsgemäß durch die Kennzeichnungsmerkmale des Anspruchs 1 gelöst.

Ein mit dar Erfindung erzielter Vorteil besteht darin, daß die Temperatur- und Fauchtigkeitsüberwachung über ein baueinheitlich kombiniertes Temperatur-FeuchteMeßkabel durchgeführt werden kann, wobei ausschließlich zwei Drähte mit einem zwischen diesen angeordneten hygroskopischen Isolator sowohl für die Erfassung einer Feuchtaänderung als auch einer Temperaturänderung erforderlich sind,so daß ein denkbar einfaches Sensorkabel für gleichzeitig mehrere Meßfunktionen gegeben ist. Das erfindungsgemäße Temperatur-Feuchte-Meßkabel ist äußerst kostengünstig herstellbar und besitzt eine hohe Lebensdauer, da die zum Einsatz gelangenden Teile auf ein Minimum reduziert sind. Außerdem ist das TemperaturFeuchte-Meßkabel sehr schmal und benötigt bei der Montage am Dampfrohr einen Dernachlässigbar geringen Platzbedarf, so daß stets eine den jeweiligen Erfordernissen angepaßte Montagepositionierung durchgeführt werden kann. Das erfindungsgemäße Temperatur-Feuchte-Meßkabel ermöglicht eine im wesentlichen Störfreie kontinuierliche Überwachung auch über größere Entfernungen. Darüber hinaus eignet sich das Temperatur-Feuchte-Meßkabel insbesondere auch für eine äußerst hohe Meßempfindlichkeit, so daß etwaige Leckstellen bereits im geringsten Ansats frühseitig erkannt werden können, Da das TemperaturFeuchte-Meßkabel als baueinheitlicher Mehrfunktionsstrang ausgebildet ist, ist eine absolute Wartungsfreiheit gegeben, da keinerlei Einzelbauteile nachsujustieren oder auszutauschen sind. Tritt im Falle einer Leckage Dampf aus der Wandung des Dampfrohres aus, so erfolgt eine Temperaturänderung, die vom Widerstandsdraht unmittelbar erfaßt wird. Gleichzeitig erfolgt eine Feuchtigkeitsanreicherung im Hygroskopisolator. Diese Feuchtigkeitsänderung im Hygroskopisolator zwischen dem Widerstandsdraht und dem Leiterdraht wird ebenfalls unmittelbar aufgrund der damit verbundenen Widerstands- bzw. Kapazitätsänderung erfaßt bzw. aufgenommen. Aufgrund der Messung der beiden unterschiedlichen Faktoren Temperatur und Feuchte ist eine hohe Sicherheit für eine zuverlässige Überwachung und Warnauslösung gegeben, da letztere ohne weiteres so konsipiert sein kann, daß ein Ansprechen ausschließlich bei einer Änderung beider Meßfaktoren ausgelöst wird.  •

Bevorzugte Ausgestaltungen und Weiterbildungen der Erfindung sind den Merkmalen der Unteransprüche, der Beschreibung und der Zeichnung zu entnehmen, wonach es vorteilhaft sein kann, das Temperatur-Feuchte-Meßkabel als Kabelschleife auszuführen und mit einem Steckerteil zu versehen, der zwei dem Widerstandsdraht zugeordnete Anschlußpole und mindestens einen Polanschluß für den Leiterdraht besitzt. Zweckmäßig können jedoch zwei Polanschlüsse für den Leiterdraht vorgesehen sein, wodurch bei der Feuchteüberwachung gleichseitig eine Drahtbruchüberwachung möglich ist.

Der Leiterdraht des Temperatur-Feuchte-Meßkabels kann vorteilhaft aus einer aus Einzeldrähten gebildeten Litze bestehen, wodurch eine hohe Flexibilität erreicht werden kann und zudem eine Bruchgefähr des Leiterdrahtes weitgehend ausgeschaltet ist. per Hygroskopisolator kann zweckmäßig als den Leiterdraht umgebende Hülle ausgeführt sein und kann zwecks hoher Beständigkeit gegen äußere Einflüsse, inebeondere auch radioaktive Strahlung, aus einem mineralischen Stoff bestehen, wobei eine Glasseidenisolation aus fertigungs- und anwendungstechnischen Gründen besonders vorteilhaft sein kann. Die Glasseidenisolation kann als Gewebeschlauch, Geflecht oder Gespinst auegeführt sein. Auch ist es möglich, den Hygroskopisolator zum Beispiel aus feuchtigkeiteaufmehmendem Zellstoff, aus Papierlagen od, dgl. herzustellen, wobei jedoch mineralische Stoffe wie Keramik oder Glas nicht nur den Vorteil einer höheren mechanischen Festigkeit sondern auch gerade eine hohe Strahlungsbeständigkeit besitzen, so daß auch bei einer Dauerstrahlungsbeanspruchung eine hohe Lebensdauer gegeben ist, weshalb das erfindungsgemäße Temperatur-Feuchte-Meßkabel auch bevorzugt im Kernkrafteektor zur Überwachung entsprechender Leitungseysteme und dgl. eingesetzt werden kann.

Die Stärke des Hygroskopisolators kann etwa im Bereich zwischen 0,01 bis 1 mm liegen. Vorzugsweise wird die Stärke des Hygroskopisolators jedoch etwa 0,1 bis 0,4 mm betragen, da hierbei zum einen ein praxisgerechter Isolationsabstand gegeben ist und zum andern eine hohe Meßempfindlichkeit vorliegt, die eine genaue Überwachung möglich macht. Der Widerstandsdraht des Temperatur-Feuchte-Meßkabels kann vorteilhaft in Form einer Schraubspirale um den den Leiterdraht umhüllenden Hygroskopisolator gewemdelt sein. Der Abstand zwischen den Wendeln kann etwa im Bereich zwischen 0,3 bis 10 mm liegen, wobei ein den praktischen Erfordernissen erforderlicher günetiger Abstand etwa 0,5 bis 4 mm sein kann. Durch eine solche Wendelausführung kann bei entsprechender Abstandsänderung der Widerstand variiert bzw. genau angepaßt werden. Zudem wird dadurch eine gewisse Eigensteifigkeit des flexiblen Temperatur-Feuchte-Meßkabels erreicht, wodurch eine bessere Handhabung wihrend der Montage möglich ist, da eine etwaige störende Lappigkeit des Kabels vermieden ist.

Weiterhin kann es insbesondere vorteilhaft sein, das erfindungsgemäße Temperatur-Feuchte-Meßkabel mit einem feuchtigkeitsdurchlässigen Außenmantel zu versehen, der den Leiterdraht, den Hygroskopisolator und den Widerstandsdraht formschlüssig umhüllt. Zweckmäßig kann ein solcher Außenmantel ebenfalls aus einem minsralischen Stoff bestehem und vortsilhaft als Glasseidsngewebesehlauch ausgeführt sein. Ein derartiger Außenmantel bewirkt zum einen eine Festigkeitserhöhung gegen äußere mechanische Beanspruchungen, so daß auch ein Einsatz unter rauhen Betriebsbedingungen ohns weiteres möglich ist. Der zweckmäßig als flexiblss Gewebe bzw. Gespinst ausgeführte schlauchförmige Außenmantel bewirkt zudem eine Potentialfreiheit, so daß praktisch keine Störungen durch Fremdspannungen, Übergangswiderstände od. dgl. das Meßsystem besinträchtigen können.

Dem erfindungsgemäßen Temperatur-Feuchte-Meßkabel wird bei der Überwachung eines Rohrleitungssystems od. dgl. sweckmäßig ein elektronischer Auswertsteil zugeordnet, der einen Temperaturmeßteil und einen Feuchtemeßteil besitzt. Weiterhin kann ein Anzeigeteil für einen etwaigen Leiterdrahtbruch der Feuehtenessung vorgesehen sein und es ist zuden möglich, einen eventuellen Widerstandsdrahtbruch ebenfalle anzuzeigen, so daß eine feuchtigkeitsund temperaturerfassende Leckageüberwachung und eine Eigenüberwachung des Meßsysteme bezüglich seiner Funktionsfähigkeit erzielt werden kann. Vortsilhaft können bei der erfindungsgemäßen Überwachungseinrichtung zwei Enden des dem Temperatur-Feuchte-Meßkabel zugehörigsn Widerstandsdrahtes den Temperaturmeßteil und zwei Enden des Leiterdrahtes dem Leiterdrahtbruchanzeigeteil im elektronischen Auswerteteil zugeordnet sein. Dem Feuchtemeßteil des elektronischen Auswertsteils wird hierbei zweckmäßig ein Leitungsteil des Leiterdrahtes und eine Teilleitung des Widerstandsdrahtes des TemperaturFeuchte-Meßkabels im Bereich des Auswerteteils zugeführt.

Zur Überwachung kann es günstig sein, das erfindungsgemäße Temperatur-Feuchte-Meßkabel im Bereich einer das Dampfrohr od. dgl. umgebenden Wärmeisolierung anzuordnen. Hierbei kann es zweckmäßig sein, die Anordnung so zu treffen, daß das Tenperatur-Feuchte-Meßkabel dicht an einer Innenseite einer Unhüllung zu liegen kommt, die die Wärmeisolierung unfaßt. Ein sich in diesem Bereich bildendes Kondensat eines eventuell austretenden Dampfes wird hier vom hygroskopischen Feuchtefühler unmittelbar erfaßt. Ebenso wird eine dabei auftretende Temperaturänderung vom Temperatur-Feuchte-Meßkabel umgehend erfaßt. Das Temperatur-Feuchte-Meßkabel kann vorteilhaft in Längsrichtung des Dampfrohres od. dgl. angeordnet sein, wodurch eine lineare Streckenüberwachung möglich ist. Dabei können auch mehrere Temperatur-Feuchte-Meßkabel auf Abstand parallel nebeneinander vorgesehen werden. Zweckmäßig känn das Temperatur-FeuchteMeßkabel im Bereich der Wärmeisolierung in einer Nut angeordnet werden, wobei die Nut nach außen durch einen im wesentlichen als Streifen ausgeführten Abdeckteil verschlossen sein kann, so daß das Meßkabel gegen Außeneinflüsse sicher abgeschirmt ist. Darüber hinaus ist es möglich, das Temperatur-Feuchte-Meßkabel praktisch ringförmig um das Dampfrohr anzuordnen und hierbei in einer entsprechenden Umfangsnut zu lagern. Dies kann zum Beispiel im Bereich von Isolationsstoßstellen und auch Rohranschlußstellen günstig sein.

Weiterhin kann es zur Erzielung störfreier Feuchteund Temperaturmessungen besonders vorteilhaft sein, den Leiterdraht im Temperatur-Feuchte-Meßkabel so auszubilden, daß er aus zwei getrennten Drahtleitersträngen besteht. Die beiden Drahtleiterstränge können parallel zueinander verlaufen. Zwischen ihnen befindet sich der Hygroskopisolator. Die Feuchtigkeitsmessung erfolgt hierbei ausschlielich über die beiden Drahtleiterstränge, so daß beide Meßsysteme so voneinander getrennt sind, daß sie sich nicht gegenseitig beeinflussen können. Es ist damit ein einfaches Sensorkabel für mehrere Meßfunktionen gegeben welches zuverlässig potentialfreie Messungen gewährleistet.

Die Drahtleiterstränge im Temperatur-Feuchte-Meßkabel sind zweckmäßig als Litzehausgeführt, die aus dünnen Kupferdrähten gebildet sind, so daß eine praktikable Flexibilität erzielt wird. Der Hygroskopisolator kann vorteilhaft als Hülle in Form eines gewebten bzw. geflochtenen Glaseidenisolationsschlauches ausgeführt sein, wobei es günstig ist, jeden einzelnen Drahtleiterstrang mit je einer Glaseidenhülle zu umgeben. Weiterhin können auch die beiden Drahtleiterstränge mit ihrem Hygroskopisolator von einem weiteren feuchtigkeitsdurchlässigen Isolator umgeben sein, welcher ebenfalls ein aus Glaseide bestehender Gewebeschlauch sein kann, in dsm die Drahtleiterstränge zusammengefaßt sind. Auf diesem die Drahtleiterstränge zusammenfassenden Isolator kann vorteilhaft der Widerstandsdraht des Temperatur-Feuchte-Meßkabels angebracht sein, wobei es günstig ist, diesen Widerstandsdraht spiral- bzw. wendelförmig gewäckelt auf dem Isolator anzuordnen. Zur Erzielung eines äußeren Gesamtabschlusses kann das Temperatur-Feuchte-Meßkabel eine Außenhülle besitzen, die feuchtigkeitsdurchlässig ist und zweckmäßig auch als geflochtener bzw. gewebter Glaseidenschlauch ausgeführt ist. Die vorbeschriebenen verschiedenen Glaseidenisolationen bewirken nicht nur eine hohe Festigkeit gegen äußere mechanische Beanspruchungen, sondern sind zudem weitgehend resistent gegenüber anderweitigen Materialien und gswährleisten zudem eine hohe Temperaturfestigkeit im Dauereinsatz.

Um die Empfindlichkeit des Feuchtemeßsystems weitgehend beeinflussen bzw. den Erfordernissen entsprechend anpassen zu können, ist es günstig, dem Leiterdraht bzw. den beiden Drahtleitersträngen einen sogenannten Grundlastwiderstand zuzuordnen, dsr sowohl für die Feuchtedetektion als auch zum Zwecke einer Drahtbruchüberwachung nützlich ist, wobei der Grundlastwiderstand zwischen den beiden Drahtleitersträngen bevorzugt an dem Ende des Temperatur-Feuchte-Meßkabels vorgesehen werden kann, welches vom Anschluß zumindest elektrisch gesehen entfernt liegt. Für den Anschluß des Temperatur-FeuchteMeßkabels kann eine vorzugsweise 4-polige Steckerbuchse vorgesehen sein, in die ein entsprechender Stecker einer Meßleitung vorzugsweise feuchtigkeits- und staubdicht eingesteckt werden kann. Darüber hinaus kann es günstig sein, einen Rückführleiter für den Widerstandsdraht vorzusehen, welcher bevorzugt durch eine Löt- oder Schweißverbindung mit dem Ende des Widerstandsdrahte s verbunden wird. Der Rückführleiter be sitzt zweckmäßig eine temperaturbeständige Isolation, die zum Beispiel aus Teflon oder auch aus Glaseide bestehen kann. Bei dieser Ausführung wird für die Temperaturmessung der Widerstand bzw. die Widerstandsänderung zwischen dem Anfang des Widerstandsdrahtes und dessen Ende erfaßt, wobei die Zurückführung von dem dem Anschluß des Temperatur-Feuchte-Meßkabels entfernt-liegenden äußersten Ende durch den Rückführleiter erfolgt. Der Rückführleiter kann außen am Temperatur-Feuchte-Meßkabel liegen und an letzterem zum Beispiel mittels elastischen bzw. gummiähnlichen Halteringen befestigt sein. Der Rückführleiter besteht vorwiegend aus einer Kupferlitze, die auch innerhalb der Außenhülle des TemperaturFeuchte-Meßkabels im wesentlichen parallel zu den Drahtleitersträngen verlaufen kann, wobei der Rückführleiter sowohl im Bereich zwischen dem Widerstandsdraht und der Außenhülle als auch im Bereich unmittelbar neben den Drahtleitersrängen liegen kann. Auch ist es möglich, den Rückführleiter des Widerstandsdrahtes zum Bei spiel aus dünnen Kupferdrähten als Flechtschlauchhülle auszuführen. In der einfachsten Anwendungsform kann das Temperatur-Feuchte-Meßkabel zum Beispiel im Bereich einer insbesondere gestopften Wärmeisolation unbeschränkt frei verlegt werden, derart, daß es sich sowohl in Längsrichtung als auch in Umfangsrichtung des Dampfrohres bzw. des Behälters oder dergleichen erstreckt. Eine bevorzugte Lage wird im Bereich zwischen der Wandung des Dampfrohres und äußeren Umhüllung der Wärmeisolation nahe der Innenfläche der Umhüllung gesehen, wobei allerdings wie bereits ausgeführt hier grundsätzlich keine Einschränkungen gegeben sind, so daß entsprechend den örtlichen Erfordernissen jeweils eine optimale Installation des TemperaturFeuchte-Meßkabels für eine einwandfreie Leckagedetektion ausgeführt werden kann.

Weiterhin kann es insbesondere vorteilhaft sein, das Temperatur-Feuchte-Meßkabel an einem Flachband anzuordnen, welches insofern flexibel ist, daß es zum Beispiel wie ein Spannband um ein Rohr bzw. dessen Wärmeisolation geschlungen werden kann. Das Flachband kann in hochwertiger Ausführung aus einem rostfreien und antimagnetischen Edelstahl bestehen und es kann einen Spannverschluß besitzen, der im wesentlichen feinstufig nachstellbar ist, so daß bei der Montage am Dampfrohr etwaige Toleranzen im Bereich der Wärmeisolierung problemlos ausgegli chen werden können, wodurch in jedem Falle ein einwandfreier Sitz des Spannbandes und damit auch des Temperatur-FeuchteMeßkabels sichergestellt ist. Die Befestigung des Temperatur-Feuchte-Meßkabels kann vorteilhaft über einen Spannklotz erfolgen, der zum Beispiel aus einem elastischen und temperaturbeständigen Silikonkautschuk hergestellt und mit einem Klemmbügel am Flachband befestigt ist. Der Spannklotz kann an der dem Flachband zugewandten Seite eine oder auch zwei Längsnuten besitzen, in denen das Temperatur-Feuchte:Meßkabel gelagert und geführt ist, wobei bei einem schleifenförmig am Flachband angeordneten Temperatur-FeuchteMeßkabel in der einen Längsnut der Vorführkabelteil und in der anderen Längsnut der Rückführkabelteil des Temperatur-Feuchte-Meßkabels sich be findet. Zur Gewährleistung einer Potentialfreiheit auch gegenüber etwaigen äußeren Einflüs sen kann zwi schen dem Spannklotz und dem Flachband eine Isolierfolie vorgesehen sein, so daß die Außenhülle des Temperatur-FeuchteMeßkabels im Bereich des Spannklotzes selbst bei einer Druckbeanspruchung nicht in eine unmittelbare Berührung mit dem Flachband gelangt. Die Isolierfolie kann aus Kapton oder dergleichen bestehen. Der Klemmbügel für den Spannklotz kann mittels Schrauben lösbar am Spannband befestigt sein. Es ist aber auch möglich, den Klemmbügel mittels Nieten oder durch Punktschweißen zu befestigen. Der Klemmbügel kann an beiden Seiten Begrenzungsstege aufweisen, deren freie Enden für ein problemloses Einstecken in eine Nut vorteilhaft schräg abgebogen bzw. gerundet sind. Die Spannklötze und Klemmbügel können etwa in fünfzehn Zentimeterabständen zueinander in Längsrichtung des Flachbandes in dessen Mittenbereich angeordnet sein. Ist das TemperaturFeuchte-Meßkabel hierbei als Kabelschleife mit einem Vorführkabelteil und einem Rückführkabelteil ausgeführt, so ist es zur Vermeidung von seitlichen Kabelausbiegungen günstig, den Vorführkabelteil und den Rückführkabelteil jeweils zwischen zwei Spannklötzen so anzuordnen, daß sie sich kreuzen. Wird das Flachband mit dem so angeordneten Temperatur-Feuchte-Meßkabel ringförmig um eine Wärmeisolation gelegt, so wölben sich die Kabelteile zwischen den Spannklötzen nicht zur Seite aus, sondern verlaufen weiterhin im wesentlichen in ihrer vorgegebenen Längsrichtung, so daß Temperatur-Feuchte-Meßkabel sicher in den Bereich der Nut in der Wärmeisolation gelangt und nicht zwischen deren äußerer Umhüllung und dem Spannband eingeklemmt wird. Vorteilhaft kann das Flachband mit dem Sensorkabel im Bereich einer Stoßstelle der Wärmeisolierung, die zum Beispiel als Kassettenisolation ausgeführt sein kann, angeordet werden. Das Flachband überdeckt dabei eine Nut, die sich im oberen Bereich der Wärmeisolierung an die Stoßstelle anschließt. Das Meßkabel gelangt dabei in den Bereich der Nut, wobei die Klemmbügel mit ihren seitlichen Begrenzungsstegen nahe den Seitenwandungen der Nut verlaufen, so daß eine Sicherung gegen ein seitliches Verschieben des Flachbandes gewährleistet ist. Der Anschluß des Temperatur-Feuchte-Meßkabels kann im Bereich der Steckerbuchse, die vorteilhaft im Bereich eines Lochs am Flachband befestigt bzw. festgeschraubt ist, in einem Anschlußblock abgedichtet sein. Dieser Anschlußblock besteht vorzugsweise aus einem feuchtigkeitsdi chten und elektrisch isolierenden Gießharz und ist vorteilhaft so gestaltet und angeordnet, daß er in Längsrichtung des Flachbandes im wesentlichen in einer Ebene mit dem Spannklotz bzw. den Klemmbügeln liegt und zudem etwa die gleiche Breite wie der Klemmbügel besitzt, so daß beim Festspannen des Flachbandes der Anschlußblock ebenfalls zentrierend in die Nut eingreift und somit zur Sicherung gegen eine etwaige Seitenversch iebung beiträgt.

Bei der Montage der Detektionseinrichtung kann zwischen dem das Temperatur-Feuchte-Meßkabel tragende Flachband und der Umhüllung der Wärmeisolierung ein Di chtungsstreifen angeordnet werden, um einen weitgehenden Dichtabschluß zu erzielen. Der Dichtungsstreifen kann dabei im Bereich der Längsseitenräder des Flachbandes verlaufen. Der Dichtungsstreifen kann aus einem Elastomer bestehen, das zum Beispiel els Schaumstoff mit geschlossenen oder auch offenen Zellen ausgeführt sein kann. Der Dichtungsstreifen kann zudem ein- oder beidseitig klebend zur Befestigung am Flachband und/oder an der Umhüllung ausgebildet sein. Die Nut im Bereich der Wärmeisolierung kann einen im wesentlichen rechteckförmigen Querschnitt haben, wobei die angrenzende Stoßstelle der Wärmeisolierung sich beliebig in der Mitte der Nut oder auch an einer Seite befinden kann. Zudem kann der Nutgrund so ausgeführt sein, daß er im wesentlichen von der Stoßstelle schräg nach außen in Richtung zum Seitenrand des Flachbandes verläuft, wodurch im Falle einer Leckage die Detektion des vorzugsweise aus der Stoßstelle austretenden Dampfanteils begünstigt werden kann. Der Schrägverlauf des Nutgrundes kann nach beiden Seiten im wesentlichen V-förmig ausgeführt sein. Es ist aber auch möglich, den Nutgrund als einzige Schräge auszuführen. Auch kann die Nut so ausgeführt sein, daß eine Abstufung gegeben ist, in welcher das Flachband im wesentlichen eingelassen bzw. versenkt gelagert werden kann, so daß nach außen am Umfang der Wärmeisolierung praktisch kein Überstand besteht.

Bei einer Überwachung eines Dampfrohres oder dergleichen empfiehlt ss sich, mehrere Überwachungsstellen vorzusehen, damit im Falle einer Leckage diese auch tatsächli ch mit großer Si cherheit frühzeitig erkannt wird. Dabei kann es günstig sein, mehrere TemperaturFeuchte-Meßkabel der verschiedenen Überwachungsstellen einem Unterverteiler zuzuführen. Zur Auswertung und Überwachung kann dem Temperatur-Feuchte-Meßkabel eine elektroni sche Meßwertverarbeitung mit Meßwertumformern für die Temperatur- und Feuchtedetektion zugeordnet werden, wobei einer solchen Meßwertverarbeitung vorteilhaft auch ein Kontrollrechnersystem zugehörig sein kann, welches vorzugsweise zwei voneinander unabhägige Mikrocomputer für die Temperatur- und Feuchtedetektion zur Datenabfrage, Störungsmeldung und eventuell auch Druckersteuerung aufweisen kann. Zudem ist für die Detektion eine Alarmanlage vorgesehen. Bei einer Anwendung in einem Kraftwerk od. dgl. kann es vorteilhaft sein, der Meßwertverarbeitung des Temperatur-Feuchte-Meßkabels eine Fließbilddarstellung oder auch eine Bildschirmanzeige zuzuordnen. Diese Anzeigegeräte können vorteilhaft in einer Warte des Kraftwerks installiert sein, welche von geschultem Personal im wesentlichen ständig besetzt ist.

Darüber hinaus kann das erfindungsgemäße TemperaturFeuchte-Meßkabel bevorzugt in einem Kernkraftwerk zur Leckagedetektion insbesondere in einem in einen Fußboden eines Raumes eingesetzten Gully angeordnet bezw. angewendet werden. Hierbei kann das Sensorkabel an einem Einsatzkörper vorgesehen werden, der im Gully gelagert ist. Dabei empfiehlt es sich, das am Einsatzkörper angeordnete Sensorkabel für eine einwandfreie Feuchtigkeitsaufnahme unmittelbar neben einer Behälterwandung des Gullys anzuordnen, so daß praktisch ein Berührungskontakt oder ein nur sehr kleiner Spalt besteht, so daß auch kleine Flüssigkeitsmengen unmittelbar vom Detektionskabel erfaßt werden, Zweckmäßig kann das Temperatur-Feuchte-Meßkabel dazu in einer Umfangsausnehmung des Einsatzkörpers sich befinden, und zwar so, daß der Außendurchmesser im Bereich des Sensorkabels etwa gleich oder etwas größer ist als der übrige Durchmesser des Einsatzkörpers, so daß das Detektionskabel in der Umfangsausnehmung weitgehend geschützt gelagert ist und dennoch ein freier Zugang für die Feuchtigkeitsaufnahme besteht. Das Temperatur-Feuchte-Meßkabel kann hierbei in mehreren Windungen, vorzugsweise zwei bis fünf Windungen, schraubgewindeartig um den Einsatzkörper verlaufen. Zur Aufnahme eines flüssigkeitsdicht kuppelbaren Steckers einer Meßleitung kann dem Temperatur-Feuchte-Meßkabel eine Steckerbuchse zugeordnet sein, die z.B. vierpolig ausgeführt ist, wobei der Anfang und das Ende des Sensorkabels hier angeschlossen sein können. Die Steckerbuchse ist dabei vorteilhaft derart isoliert im Einsatzkörper angeordnet, daß ein feuchtigkeitsdichter Abschluß insbesondere im Anschlußbereich des Detektionskabels gegeben ist. Die Steckerbuchse und der Einsatzkörper mit dem integrierten Temperatur-FeuchteMeßkabel bilden somit im wesentlichen eine einzige kompakte Baueinheit, die den praktischen Erfordernissen sowohl während der Montage als auch während des späteren Dauerbetriebs zuverlässig genügt. Die Steckerbuchse kann bei einer Ausführung im wesentlichen im Mittenbereich des Einsatzkörpers über radiale Stützstreben gehalten sein. Zwischen den Stützstreben befinden si ch große Freiräume für den Durchgang einer in den Gully gelangenden größeren Flüssigkeitsmenge. Der Einsatzkörper kann dabei nach unten konisch verjüngt ausgeführt sein und an seinem oberen Bereich einen schwenkbaren Handhabungsbügel insbesondere zum leichten Herausnehmen aus dem Gully besitzen. Der Einsatzkörper kann aus Kunststoff bestehen, es kann auch Edelstahl verwendet werden. Das Temperatur-FeuchteMeßkabel kann bei dieser Ausführungsform von der Außenwandung des Einsatzkörpers zu der im Mittenbereich angeordneten Steckerbuchse unter einer der radialen Stützstreben in deren Querschnittsbereich in einer entsprechenden Aussparung verlaufen, so daß in jedem Falle eine gsschützte Lagerung sichergestsllt ist. Bei einer anderen bevorzugten Ausführung kann der Einsatzkörper mit dem Sensorkabel in einem als Rohr ausgeführten Behälterteil gelagert sein. Dieses rohrförmige Behältsrteil kann an der Innenfläche eines in den Gully einsetzbaren Hinges befestigt sein, so daß eine außermittige Anordnung dss Einsatzkörpers gegeben ist. Im Befestigungsbereich des rohrförmigen Behälterteils am Hing kann eine muldenförmige Flüssigkeitszuführung vorgesehsn sein, durch die eine etwaige Leckflüssigkeit zwangsgeführt in den Rohrbehälterteil und damit an das am Einsatzkörper befindliche Temperatur-Feuchte-Meßkabel herangeführt wird. Der Einsatzkörper ist im rohrförmigen Behälterteil weitgehend spielfrei gelagert. Eine etwaige von oben einfließende Flüssigkeit kann unten aus dem Rohrbehälterteil nach dem Passieren des Sensorkabels frei abfließen. Der Ring kann eine Umfangsnut mit einem 0-Hing besitzen, so daß beim Einsetzen in den Gully am Außenumfang des Hinges ein dichter Abschluß gegeben ist und sine etwaigs Flüssigkeit nicht außen am Ring vorbeifließen kann. Selbstverständlich kann auch bei dieser Gully-Überwachung nicht nur eine Feuchteänderung, sondern auch eine Temperaturänderung erfaßt wsrden, so daß auch hier zwei Parameter für sine eindeutige und si chere Leckagedetektion gewährleistet ist, wenn zum Beispiel aus einem Rohrsystem, einem Behälter oder dergleichen Flüssigkeit austritt und in den Gully gelangt, wobei bereits geringe Temperaturunterschieds zwischen der Leckageflüssigkeit und der Raumtemperatur erfaßt werden. Der erfindungsgemäßen Gully-Überwachung mit dem vorliegenden TemperaturFeuchte-Meßkabel kann zweckmäßig eine Meßwertverarbeitung vorzugsweise mit Meßumformern, Auswerteelektronik, Kontrollsystem ( Drucker), Alarmgeber und dgl. zugeordnet werden. Besondere Bedeutung kommt der Gully-Überwachung an den Stellen zu, die von Personen normalerweise nicht aufgesucht werden dürfen. Dies sind z.B. in einem Kernkraftwerk bestehende Räumlichkeiten, die z.B. aufgrund einer radioaktiven Strahlungsgefährdung aus Sicherheitsgründen nur in Ausnahmefällen bzw. in großen Zeitabständen betreten werden dürfen. Durch das vorgeschlagene Gully-Überwachungssystem ist eine kontinuierliche Fernüberwachung möglich, die eine hohe Betriebssicherheit mit zuverlässiger Dauerfunktion besitzt und potentialfrei arbeitet, so daß äußere Störeinflüsse praktisch vermieden sind. Das vorgeschlagene Gully-Überwachungssystem ist zudem relativ kostengünstig herzustellen und zu installieren, so daß sämtliche in Frage kommende Gullys im Berei ch einer zu überwachenden Kraftwerkseinrichtung unter Vermeidung eines unverhältnismäßig hohen Kostenaufwands erfaßt werden können, zumal nach der Installation des Gully-Überwachungssystems etwaige Folgekosten praktisch ni cht auftreten bzw. auf ein Minimum reduziert sind. Es sei noch erwähnt, daß zur Feuchtemessung die beiden Drahtleiterstränge im Temperatur-Feuchte-Meßkabel einem Feuchtemeßteil zugeführt werden, über den eine Widerstandsäderung im M-Ohm Bereich zwi schen den Drahtleitersträngen am Hygro skopisolator erfaßt wird. Darüber hinaus ist es insbesondere günstig, daß im Bedarfsfalle wahlweise eine solche elektrische Spannung an den Widerstandsdraht des Temperatur-Feuchte-Meßkabels angelegt werden kann, daß der Widerstandsdraht als elektrisches Hei zelement wirkt. Durch diese erfindungsgemäße Maßnahme kann mit ein und demselben Sensorkabel auch eine Trocknung etwaiger Feuchteanreicherungen erzielt werden, was z.B. zur Durchführung einer Kontrollmessung bei einer vorausgegangenen Signalgabe vorteilhaft sein kann. Es ist damit die Möglichkeit gegeben, ohne zusätzliche Mittel das Detektionssystem absolut trocken zu halten,um einwandfreie Meßdurchführungen zu gewährleisten. Zudem wird dadurch in der Praxis ein wesentlicher wirtschaftlicher Vorteil erzielt, da eine derartige Trocknung über den als Heizelement wirkenden Widerstandsdraht im Temperatur-Feuchte-Meßkabel sowohl letzteres selbst als auch dessen Umgebung in ausgesprochen kurzer Zeit von Feuchtigkeit befreit bzw. getrocknet werden kann, da eine derartige Trocknung schon in einigen Minuten durchgeführt werden kann, während eine sonstige Trocknung ohne Widerstandsdrahtheizung durchaus einige Tage in Anspruch nehmen kann.

Bevorzugte Ausgestaltungen und Weiterbildungen sowie weitere Vorteile und Einzelheiten der Erfindung sind den Merkmalen der Unteransprüche, der nachfolgenden Beschreibung und der Zeichnung zu entnehmen, die in schematischer Darstellung bevorzugte Ausführungsformen als Beispiel zeigt. Es stellen dar:

FIG. 1 eine vergrößerte Teilschnittansicht eines erfindungsgemäßen Temperatur-Feuchte-Meßkabel s,

FIG. 2 eine Teilschnittansicht eines Dampfrohres mit einem als Schleife ausgeführten TemperaturFeuchte-Meßkabel,

FIG. 3 eine Teils chnittansi cht eines anderen Dampfrohres mit einem in einer Umfangsnut angeordneten Temperatur-Feuchte-Meßkabel und

FIG. 4 ein Schaltschema einer erfindungsgemäßen Überwachungseinrichtung mit einem Temperatur-FeuchteMeßkabel und einem Auswerteteil,

FIG. 5 eine Seitenansicht eines Teils eines erfindungsgemäßen Temperatur-Feuchte-Meßkabels in starker Vergrößerung,

FIG. 6 eine weitere Ausführung eines Temperatur-FeuchteMeßkabels in einer Ansicht ähnlich FIG. 1,

FIG. 7 eine Gesamtansicht eines frei verlaufenden Temperatur-Feuchte-Meßkabels,

FIG. 8 eine Unteransicht eines Detektionsspannbandes,

FIG. 9 eine Seitenansicht des Detektionsspannbandes gemäß FIG. 4,

FIG. 10 eine vergrößerte Schnittdarstellung des Detektionsspannbandes gemäß FIG. 4,

FIG 11 das Detektionsspannband gemäß FIG. 4-6 in einer kreisförmig gebogenen Darstellung wie bei einer Montageanordnung um ein isoliertes Dampfrohr od. dgl.,

FIG. 12 eine Schnittansicht des Detektionsspannbandes der FIG. 7,

FIG. 13 eine Teilschnittdarstellung eines isolierten Dampfrohres mit einem Detektionsspannband gemäß FIG. 4-8,

FIG. 14 eine andere Teilschnittdarstellung eines Dampfrohres ähnlich der FIG. 9,

FIG. 15 eine weitere Teilschnittdarstellung eines Dampfrohres ähnlich den Figuren 9 und 10,

FIG.16 eine Prinzipdarstllung einer gesamten Überwachungseinrichtung zur Leckage-Detektion,

FIG. 17 eine Prinzipdarstellung der erfindungsgemäßen Überwachungseinrichtung zur Leckagedetektion mit einer Computerauswertung,

FIG. 18 eine Darstellung eines Temperatur-Meßumformers der Überwachungseinrichtung zur Leckagedetektion,

FIG. 19 eine Darstellung eines Feuchte-Meßumformers der Überwachungseinrichtung zur Leckagedetektion,

FIG.20 ein Einstellbeispiel für den Feuchte-Meßumformer,

FIG. 21 ein Einstellbeispiel für den Temperatur-Meßumformer,

FIG.22 einen Gully-Detektor der erfindungsgemäßen Überwachungseinrichtung mit einem Temperatur-FeuchteMeßkabel,

FIG.23 eine weitere Ausführungsform eines Gully-Detektors der Überwachungseinrichtung,

FIG. 24 eine Systemdarstellung der Überwachungseinrichtung zur Leckagedetektion mit Gullyüberwachung.

Das in der Zeichnung dargestellte Temperatur-FeuchteMeßkabel 1 dient der Überwachung eines Dampfrohres 2 od, dgl., wobei im Falle eines Lecks aufgrund des austretenden Dampfes Feuchtigkeits- und Temperaturänderungen erfaßt werden. Hierzu weist das erfindungsgemäß verblüffend einfache Temperatur-Feuchte-Meßkabel 1, das somit ein mehrfunktionales und baueinheitlich kombiniertes Sensorkabel ist, einen mittleren Leiterdraht 3 auf, der vorzugsweise aus Kupfer besteht und als aus dünnen Einzeldrähten 4 gebildete Litze ausgeführt ist.

Der Leiterdraht 3 des Temperatur-Feuchte-Meßkabels 1 ist von einem feuchtigkeitsaufnehmenden Hygro skopi solator 5 umgeben, der beim vorliegenden Ausführungsbeispiel als schlauchförmige Hülle ausgeführt ist und aus einer Glasseidenisolation besteht, die vorteilhaft fein versponnene oder verwebte Glasseidenfüden aufweist. Zur Erzielung einer hohen Meßempfindli chkeit beträgt die Wandstärke des Hygroskopisolators 5 im vorliegenden Falle etwa 0, 3 mm. Es ist aber auch möglich, die Stärke des Hygroskopisolators 5 etws dünner oder auch etwas dicker auszuführen, um eine Anpassung an unterschiedliche Praxisanforderungen insbesondere auch bezüglich der Meßempfindlichkeit zu ermöglichen.

Parallel zum Leiterdraht 3 verläuft in dessen Längsri chtung ein dünner Widerstandsdraht 6, der praktisch wendelförmig um den Hygroskopisolator 5 gewickelt ist, so daß durch letzteren eine konstant gleichbleibende Abstandhaltung zum Leiterdraht 3 gegeben ist. Der Widerstandsdraht 6 wird zweckmäßig so gewickelt, daß die Abstände zwischen den Wendeln etwa bei 1 mm liegen, wobei es aber auch im Rahmen der Erfindung liegt, die Wendelabstäde größer oder kleiner auszuführen. Aufgrund der Wendelausführung des Widerstandsdrahtes 6 weist das Temperatur-Feuchte-Meßkabel 1 eine gewisse Eigensteifigkeit auf und es wird zudem der Vorteil einer umfangsseitig weitgehend dichten bzw. engen Meßerfassung erzielt.

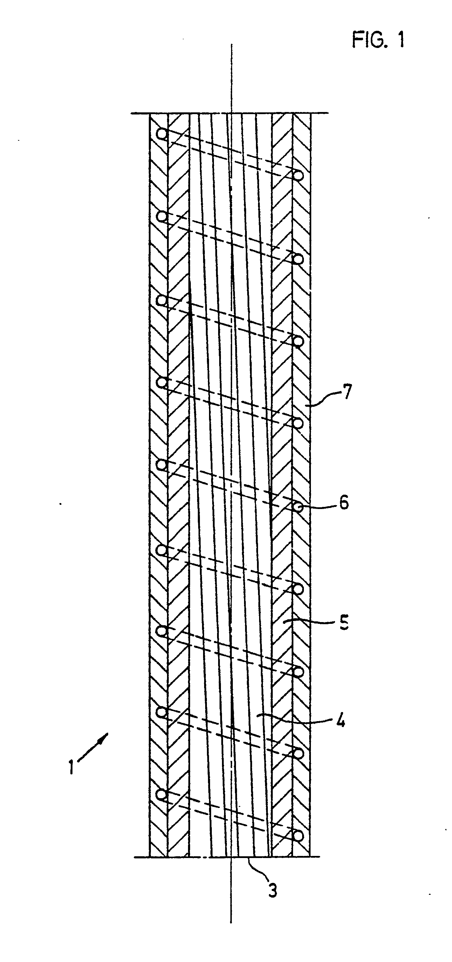

Weiterhin iet der Fig. 1 zu entnehmen, daß das Temperatur-Feuchte-Meßkabel 1 einen Außenmantel 7 besitzt, der dsn Leiterdraht 3, den Hygroskopisolator 5 und den Widerstandedraht 6 schlauchföralg umschließt. Dieser Außenmantel 7 besteht zweckmäßig ebenfalls aus einem mineralischen Stoff und ist beim vorliegenden Ausführungsbsispiel aus Glasseidenfäden hergestellt wordsn, die zu einem mechanisch festen, aber dennoch feuchtigkeitsdurchlässigen Gewebeschlauch verarbeitet sind.

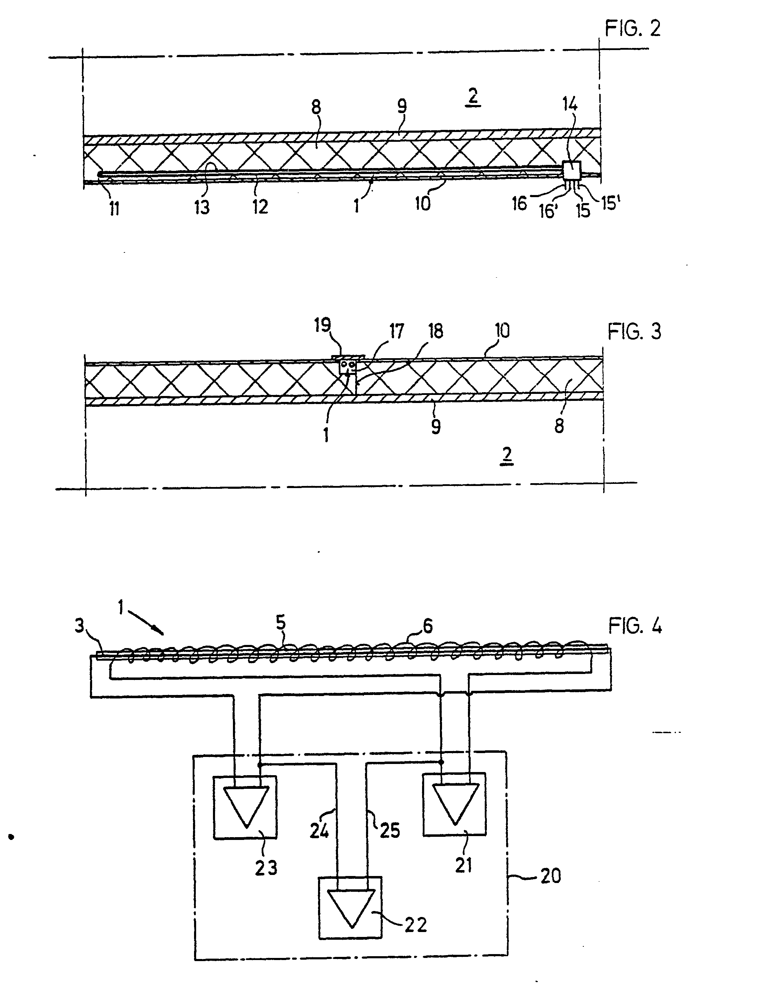

Beim Ausführungsbeispiel der Fig. 2 ist das TemperaturFeuchte-Meßkabel 1 in Lägsrichtung des Dampfrohres 2 im Bereich ein er Wärmeisolierung 8 angeordnet, die die Wandung 9 des Dampfrohres 2 umgibt. Die Anordnung ist so getroffen, daß das Temerpatur-Feuchte-Meßkabel 1 sich nahe der Innenseite einer äußeren Umhüllung 10 befindet, die als Blechmantel oder auch Kunststoffmantel ausgeführt sein kann. Das Temperatur-Feuchte-Meßkabel 1 ist hierbei als Kabelschleife 1 1 ausgeführt, wobei ein umgelenktes Rückführungskabelteil 12 im wesentlichen parallel zu einem Vorführungskabelteil 13 verläuft, die mit einem Ende an einem Steckerteil 14 angeschlossen sind, der aus der Umhüllung 10 herausragt. Der Steckerteil 14 besitzt zwei Anschlußpole 15, 15' für den Widerstandsdraht 6 und zwei Polanschlüsss 16, 16' für den Leiterdraht 3.

Bei dem in der Fig. 3 därgestellten Ausführungsbeispiel ist das erfindungsgemäße Temperatur-Feuchte-Meßkabel 1 in einer Nut 17 gelagert, die als radiale Umfangsnut ausgsführt ist und sich im Bereich einer Stoßstells 18 der Wärmeisolierung 8 befindet. Die Nut 17 ist außen durch einen flachbandförmigen Abdecktsil 19 vsrschlos sen, so daß das Temperatur-Feuchte-Meßkabel 1 sicher abgeschirmt ist. Auch hierbei ist das Temperatur-FeuchteMeßkabel 1 in der Nut 17 schleifenförmig angeordnet, so daß eine Vor- und eins Rü ckführung gegeben ist.

Der Ausführung gemäß Fig. 4 ist ein prinzipieller Schaltungsaufbau der Übsrwachungseinrichtung zu entnehmen. Dabei ist ein vorzugsweise elektronischer Auswerteil 20 vorgesehen, der einen Temperaturmeßteil 21, einen Feuchtemeßteil, 22 und einen Leiterteilbruchanzeigeten (Feuchte) 23 umfaßt. Es ist ersichtlich, daß zwei Enden dss dem Temperatur-Feuchte-Meßkabel 1 zugehörigen Widerstandsdrahtes 6 dem Temperaturmeßteil 21 zugeführt sind. Die zwei Endsn des Leiterdrahtes 3 sind dem Leiterdrahtbruchanzsigeteil 23 zugeführt. Dem Feuchtemeßteil 22 ist ein dem Leiterdraht 3 zugehöriger Leitungsteil 24 und eine dem Widerstandsdraht 6 zugehörige Teilleitung 25 zugeordnet. Der Leitungsteil 24 und die Teilleitung 25 sind zweckmäßig im Bereich des Auswertsteils 20 an entsprechenden Stellen abgegriffen bzw. angeschlossen.

Das in der Zeichnung dargestellte Temperatur-FeuchteMeßkabel 101 ist Teil einer Überwachungseinrichtung zur Leckagedetektion an z.B. Flüssigkeit oder Dampf aufnehmenden bzw. beinhaltenden Behältern od. dgl.. Es ist als baueinheitlich mehrfunktionales Sensorkabel ausgeführt und besitzt zwei als Leiterdraht 102 wirkende Drahtleiterstränge 103, 104. Letztere bestehen aus dünnen Kupfereinzeldrähten, so daß sis als sogenannte Litzen ausgeführt sind, die eine gewisse Flexibilität beinhalten. Bside Drahtleitersträge 103, 104 sind je einzeln mit einem Hygroskopisolator 105 versehen, der beim vorliegenden Ausführungsbeispiel als geflo chtene Glasseidens chlauchhülle ausgeführt ist. Die beiden Drahtleiterstränge 103, 104 sowie der Hygroskopisolator 105 befinden sich in einem weiteren Isolator 106, welcher ebenfalls feuchtigkeitsdurchlässig ist und hier als schlauchförmig umhüllendes Glasseidengewebe ausgeführt ist. Um den I solator 106 ist wendelförmig bzw. schraubengewindeartig ein Widerstandsdraht 107 gewickelt, der zur Temperaturmessung dient, während die beiden Drahtleiterstränge 103, 104 mit dem Hygroskopisolator 105 für die Feuchtemessung bestimmt sind, wobei die Drahtleitersträge 103, 104 einem Feuchtemeßteil zugeführt sind, über den eine Widerstandsäderung im M-Ohm-Bereich zwischen den Drahtleitersträgen 103, 104 erfaßt wird. Temperatur und Feuchte werden somit im erfindungsgemäßen Detektionskabel 101 mit Sensorsn erfaßt, dis vollständig voneinander getrennt sind, so daß ksinerlei gegenseitige Beeinflussung auftreten kann und in jedem Falle potentialfreie Meßergebnisse erzielt werden. Der Widerstandsdraht 107, der einem Temperaturmeßteil zugehörig ist, kann im Bedarfsfalle auch an eine derartige elektrische Spannung gelegt werden (Umschaltung), daß der Widerstandsdraht 107 als elektrisches Heizelement arbeitet. Dadurch ist es auf einfache Weise möglich, etwaige Feuchteanteile im Temperatur-Feuchte-Meßkabel 101 und in dessen Umgebung durch Trocknung schnell zu beseiti gen. Außen besitzt das Temperatur-Feuchte-Meßkabel 101 einen Außenmantel 108, der aus einem schlauchförmig umhüllenden Glasseidengewebe besteht und die vorgenannten Drahtleiterstränge 103, 104, deren Hygroskopisolator 105, den Isolator 106 und den Wi derstandsdraht 107 umfaßt. Am Außenmantel 108 liegt ein Rückführleiter 109 an, der mit entsprechenden elastischen Halteringen od. dgl. am Außenmantel 108 festgelegt sein kann. Der Rückführleiter 109 ist außen mit einer temperaturbeständigen Teflonisolation 110.versehen, wobei auch Silikonkautschuk oder Glasseide vorgesehen werden kann. Der Rückführleiter 109 kann mittels einer Löt- oder Schweißverbindung mit dem Ende des Widerstandsdrahtes 107 bei z. B. nicht zurückgeführtem Temperatur-Feuchte-Meßkabel 101 zur Rückleitung verbunden werden.

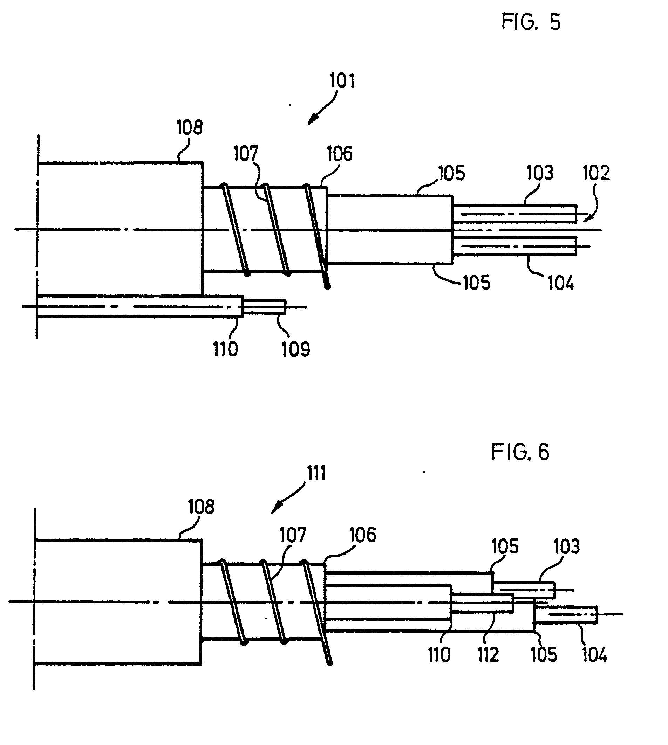

Das in der FIG. 6 dargestellte Temperatur-Feuchte-Meßkabel 111 ist im wesentlichen wie das vorstehend beschriebene Temperatur-Feuchte-Meßkabel 101 ausgeführt. Deshalb besitzen die übereinstimmenden Teile die glei chen Bezugszeichen. Der wesentliche Unterschied besteht darin, daß beim Temperatur-Feuchte-Meßkabel 111 der Rückführleiter 112 parallel neben den Drahtleitersträgen 103, 104 innerhalb des schlauchförmig umhüllenden Isolators 106 verläuft. Es liegt zudem im Rahmen der Erfindung, den Rückführleiter 112 z.B. als Kupfergeflecht unter dem Isolator 106 vorzusehen.

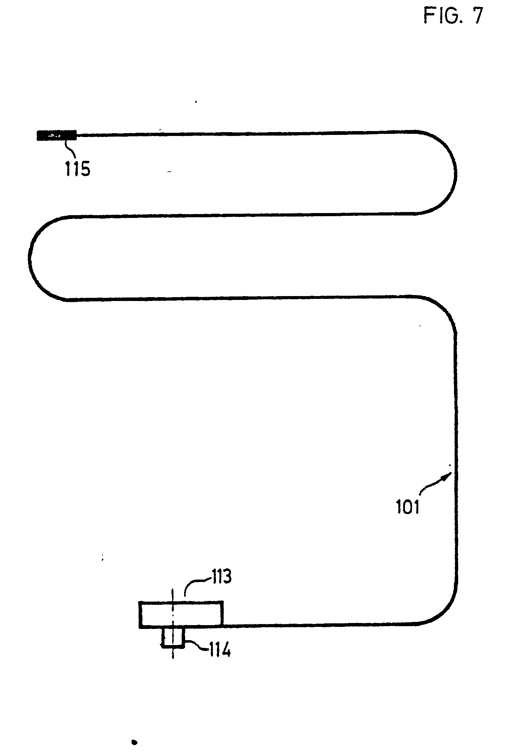

Gemäß der FIG. 7 kann das Temperatur-Feuchte-Meßkabel 101, 111 im wesentlichen ohne irgendwelche besonderen Einschränkungen weitgehend frei verlegt werden, so daß eine beliebige Anordnung entlang einer Rohrleitung vorzugsweise in einer gestopften Isolation erfolgen kann.

An einem Ende besitzt das Temperatur-Feuchte-Meßkabel 101 einen Anschlußblock 113 mit einer mehrpoligen Steckerbuchse 114. Diese ist in dem Anschlußbleck 113 feuchtigkeitsdicht elektrisch isoliert gelagert, da der Anschlußblock 113 aus einem entsprechenden Gießharz besteht, welches vorzugsweise temperaturbeständig wie auch elastisch sein kann. Am anderen Ende weist das Temperatur-Feuchte-Meßkabel 101 einen Grundlastwiderstand 1 15 auf, der zwischen den der Feuchtedetektion dienenden Drahtleitersträgen 103, 104 angeordnet ist und zudem für eine Drahtbruchüberwachung mit herangezogen werden kann.

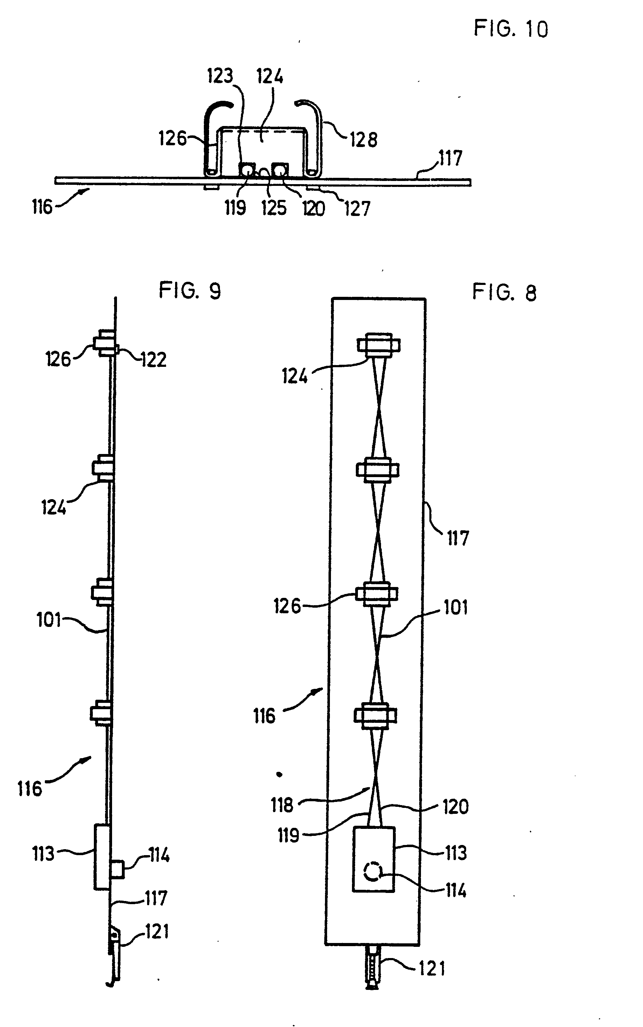

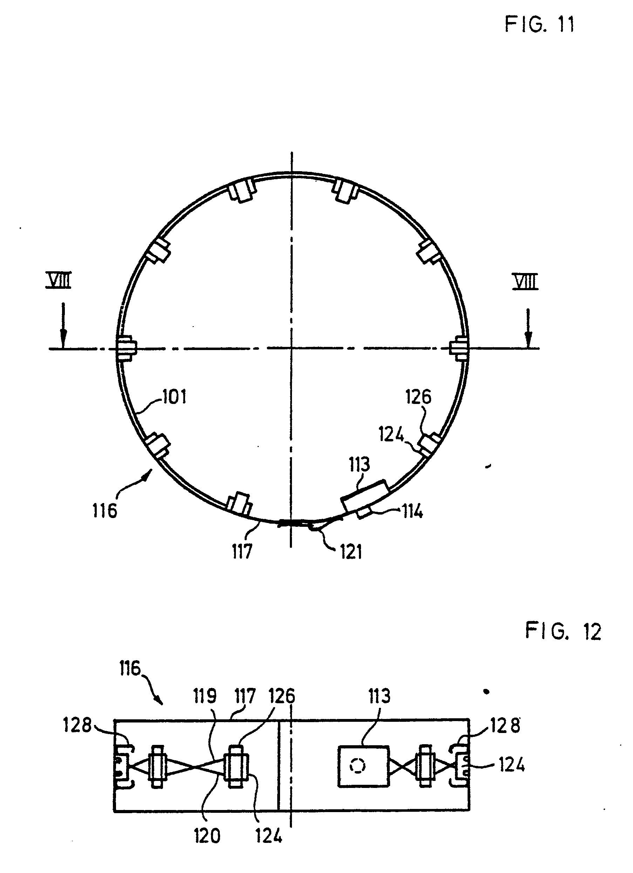

Bei dem in den Fig. 8-15 ersichtlichen Detektionsspannband 1 16 ist das Temperatur-Feuchte-Meßkabel 101 als an einem Flachband 1 17 festgelegte Kabelschleife 1 18 ausgeführt, die einen Vorführkabelteil 1 19 und einen Rückführkabelteil 120 aufwei st. Das Flachband kann aus einem etwa 1,5 mm dünnen Edelstahlblech bestehen, an dessen einem Ende für einen Schnellverschluß ein schwenkhebelbetätigbarer Spannverschluß 121 angeordnet ist,der über einen Gewindeteil für einen Toleranzausgleich feinstufig nachgestellt werden kann und am anderen Endbereich des Flachbande 117 in ein Widerlager 122 eingreift. Das Temperatur-Feuchte-Meßkabel 101 ist mit seinem Vorführkabelteil 119 und seinem Rückführkabelteil 120 in Längsnuten 123 mehrerer Spannklötze 124 gelagert. Die Spannklötze 124 sind im wesentlichen in gleichen Abständen von etwa 50 cm am Flachband 1 17 befestigt und bestehen in einer bevorzugten Ausführung aus gummielastischem Silokonkautschuk, der zu seiner guten elektrischen Isoliereigens chaft zudem eine hohe Dauertemperaturbeständigkeit aufweist. Unter den Spannklotz 124 kann zusätzlich jeweils noch eine z.B. aus Kapton bestehende Isolierfolie 125 angeordnet sein, so daß zwischen dem Temperatur-Feuchte-Meßkabel 101 und dem Flachband 1 17 zur Abschirmung gegen äußere Störeinflüsse für eine auch in-sofern potentialfreie Meßdurchführung eine hochwertige Isolation gegeben ist. Die Spannklötze 124 sind jeweils mit einem Klemmbügel 126 befestigt, der mittels Schrauben 127 am Flachband 117 lösbar angeordnet ist. Jeder Klemmbügel 126 besitzt an beiden Seiten vom Flachband 117 abstrebende Begrenzungsstege 128, deren freie Enden bogenförmig gerundet sind. Es ist zudem zu erkennen, daß der Vorführkabelteil 119 und der Rückführkabelteil 120 jeweils im Bereich zwischen zwei Spannklötzen 124 so verlaufen, daß sie sich kreuzen,

8

Dadurch wird bei einem Rundbiegen des Flachbandes 117 zu einem Kreis vermieden, daß sich der Vorführkabelteil 119 und der Rückführkabelteil 120 nach außen bogenförmig wegbiegen. Auch ist zu erkennen, daß die Breite des Anschlußblocks 1 13 im wesentli chen gleich der Breite der Klemmbügel 126 ist und mit diesen in Längsrichtung des Flachbandes 117 gesehen in der gleichen Mittenebene liegt. In dem vom Anschlußblock 113 entfernt liegenden Spannklotz 124 ist zwischen den Längsnuten 123 eine Quernut ausgebildet, in der die Umlenkung des Temperatur-Feuchte-Meßkabels 101 erfolgt.

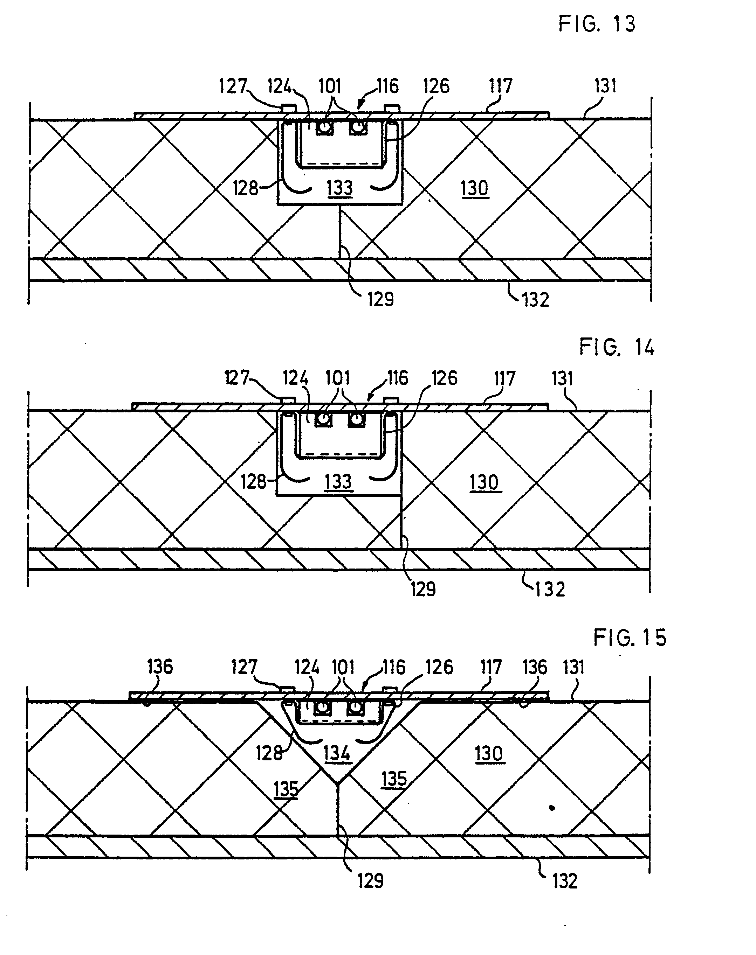

In den Fig. 13-15 i st zu erkennen, daß das TemperaturFeuchte-Meßkabel 101 im wesentlichen im Bereich einer Stoßstelle 129 von zusammengesetzten Wärmeisolierungen 130 im wesentlichen in der Ebene der äußeren Umhüllung 131 liegt. Die Wärmeisolierung 130 umschließt die Wandung 132 eines Dampfrohres. In FIG. 13 ist die Stoßstelle 129 in der Mitte des Grundes der rechteckförmigen Nut 133 angeordnet. In FIG.14 liegt die Stoßstelle 129 an einer Seite der Nut 133, während in FIG. 15 die Stoßstelle 129 wiederum in die Mitte der Nut 134 einmündet, welche hier im wesentlichen dreieckförmig ausgebildet ist, so daß Schrägteile 135 gebildet sind. Außerdem ist hier zwischen dem Flachband 117 und der Umhüllung 131 der Wärmeisolierung 130 an beiden Seiten des Flachbandes 117 ein Dichtungsstreifen 136 vorgesehen. Der Klemmbügel 126 ist mit seinen seitlichen Begrenzungsstegen 128 so ausgeführt, daß letztere annähernd formschlüssig in die Nut 133, 134 eingreifen und somit das Detektionsspannband 116 bei der Montage und auch darüber hinaus sicher gegen eine Verschiebung zur einen oder anderen Seite arretieren. Durch die Ausführung der Schrägteile 135 kann der Vorteil einer kostengünstigen Herstellung bei der Anfertigung der Wärmeisolierung 130 erreicht werden, wobei es im Rahmen der Erfindung liegt, die Wärmeieolierung bzw. die Nut so zu gestalten, daß der Nutgrund ausschhließlich von einem einzigen Schrägteil gebildet ist, während die andere Nutbegrenzung durch die sich bis zur äußeren Umhüllung 131 erstreckende Stoßstelle 129 gebildet wird. Auch hierbei kann eine kostengünstige Herstellung erreicht werden.

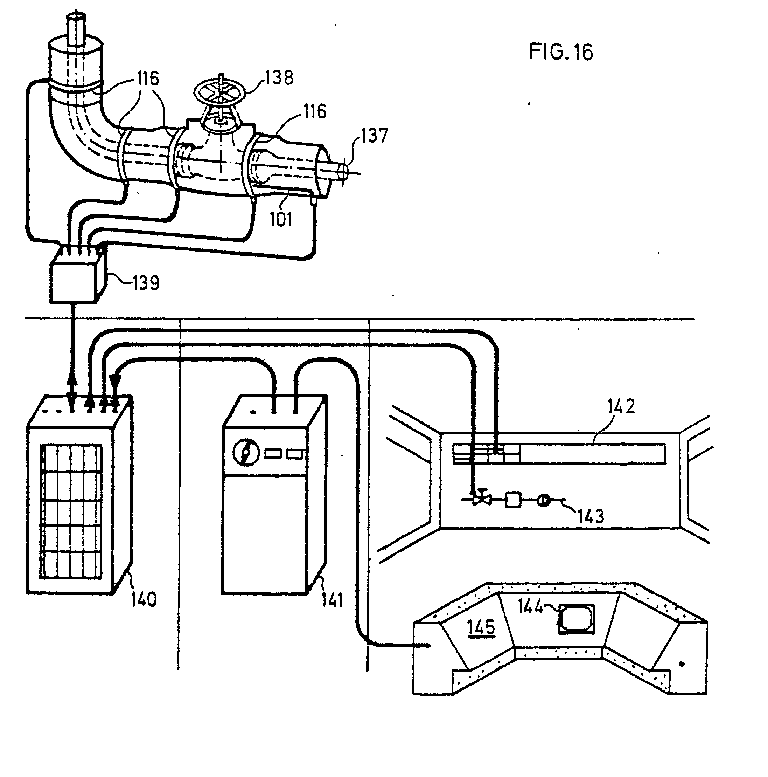

Die FIG. 16 zeigt eine erfindungsgemäße Überwachungseinrichtung mit fünf Meßstellen, wovon vier Meßstellen durch Detektionsspannbänder 116 gebildet sind, die an Stoßstellen einer Wärmeisolierung 130 eines Dampfrohres 137 angeordnet sind' wobei zwei Detektionsspannbänder 116 sich im Bereich von Anschlußflanschen eines Ventils 138 befinden. Eine Meßstelle ist als frei verlegtes Sensorkabel parallel zur Längsrichtung des Dampfrohres 137 im Bereich der Wärmeisolierung 130 vorgesehen. Von den fünf am Dampfrohr 137 vorgesehenen Temperatur/Feuchte-Sensoren 101 bzw. 116 führen Meßleitungen zu einem Unterverteiler 139, in dem diese Sensorgruppe zunächst zusammengefaßt ist.

Von hier aus besteht eine Verbindung zu einer elektroni schen Meßwertverarbeitung 140, welcher Meßwertumformer und ein Kontrollrechnersystem 141 mit zwei Mikrocomputern zur Datenabfrage, Störungsmeldung, Meßauswertung (Drucker) zur Temperatur- und Feuchteerfassung zugeordnet ist. Die Mikrocomputer arbeiten voneinander unabhägig. Außerdem ist der Meßwertverarbeitung 140 in einer Warte eines Kernkraftwerks eine Alarmanlage 142 zugeordnet und es ist zudem eine Fließbilddarstellung 143 sowie eine Bildschirmdarstellung 144 zur städigen bzw. beliebig abfragbaren Kontrolle vorgesehen. Die Meßergebnisse können über einen Drucker 145 schriftlich festgehalten werden.

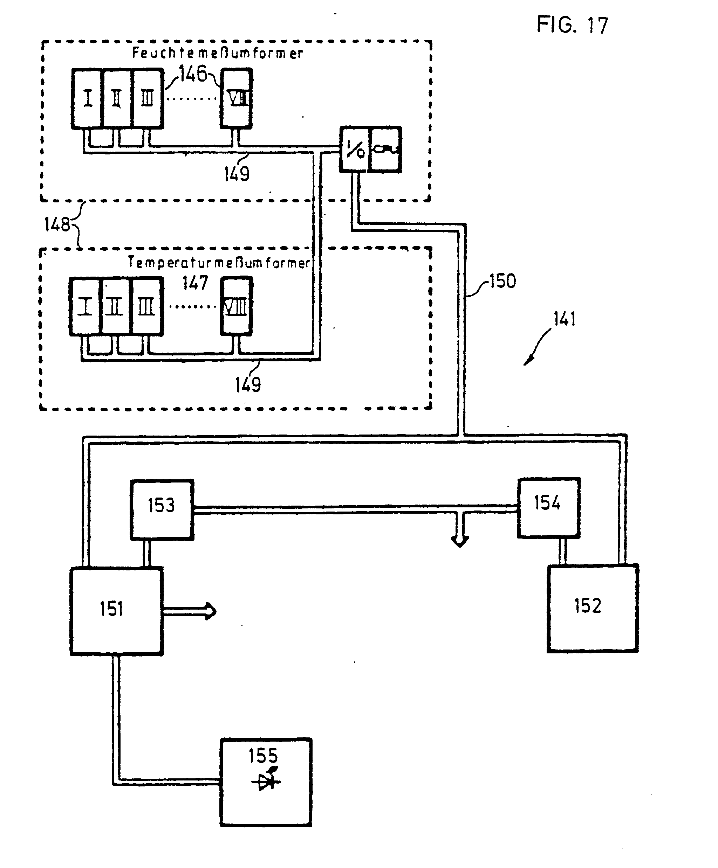

Die FIG. 17 verdeutlicht den Aufbau mit der Computerauswertung mit dem Feuchtemeßumformer 146 und dem Temperaturmeßumformer 147 sowie dem Kontrollrechnersystem 141. Hierbei kommt der Betriebssicherheit und der Überschaubarkeit eine wesentliche Bedeutung zu. Alle eingesetzten Rechner und Meßumformer sind in C-Mos-Technik ausgeführt. Dies gewährlei stet eine geringe Stromaufnahme und einen großen Störabstand. Die Verbindungen innerhalb eines 19-Zoll Einbaurahmens 148 sind auf einer großen Verdrahtungsplatine ( Backplane) 149 zusammengefaßt. Der Einbaurshmen 148 umfaßt acht Feuchtemeßumformer 146, acht Temperaturmeßumformer 147, Mikrocomputer für die Überwachung und zyklische Funktionsprüfung der sechzehn Meßumformer sowie eine Datenaufbereitung zur seriellen, bidirektionalen Übertragung auf einem Zwei-Draht-Datenbus 150. Das Kontrollrechnersystem 141 ist komplett redundant aufgebaut. Es können bis zu 1024 Meßumformer über bidirektionalen Zwei-Draht;Datenbus angeschlossen werden. Das Kontrollrechnersystem 141 weist zwei voneinander unabhängige Mikrocomputer ( 100% redundant) zur Dnatenanbfrage Störungsmeldung, Druckersteuerung etc. auf. Vom Rechner 151 sind Abgänge zum Drucker, Tastenfeld, Sammelmeldung, Anzeige Absolutwert und Anzeige Meßstellennummer vorgesehen. Zur Umschaltung bei einem Ausfall eines der beiden Rechner 151, 152 von einem zum anderen Rechner sind sogenannte 'WatchDog' 153, 154 vorgesehen, zwischen denen ein pfeilförmig angedeuteter Abgang zu einer Systemstörung bzw. Systemstöranzeige vorgesehen ist. Der Rechner 151 ist mit einem Rohrleitungsschema 155 verbunden. Zur Dokumentation aller Meldungen, Abfragen, Störungen etc. wird ein alphanumerischer Drucker eingesetzt. Um bis zu 1000 Meßstellen anwählen zu können, ist ein Sechzehn-Tastenfeld vorhanden. Dazu können zwei Anzeigenfelder mit je zwei 4-stelligen 7-Segmentanzeigen eingesetzt und eine Ansteuerschaltung für ein Anzeigentableau mit bis zu 1000 Leuchtdioden vorgesehen werden.Es liegt jedoch im Rahmen der Erfindung, das Kontrollrechnersystem 141 auch in einer anderen bzw. kleineren Ausführung zu erstellen bzw. anzupassen, wobei auch insbesondere eine Integration in bereits vorhandene Überwachungsanlagen bei einer nachträglichen Installation des erfindungsgemäßen Überwachungssystems berücksichtigt werden kann.

Bei der Meßwertverarbeitung erfüllt die Zentraleinheit mit zwei unabhägigen Rechnern in Parallelschaltung und gegenseitiger Plausiblitätskontrolle die folgenden wichtigsten Funktionen: Steuerung der Prüfzyklen, Überwachung der Unterrechner, Überwachung des Datenbusses, Ansteuerung Drucker und Anzeigen, Tasteneingabe, Selbstüberwachung, Selbsttest. Zur Meßstellenanwahl und Signalisierung bietet das System folgende Möglichkeiten, welche gleichzeitig in Betrieb sein können: Signalisierung der Grenzwertausgänge optisch, akustisch und Druckerausgang; Handanwahl einer Meßstelle zur Trendbeobachtung, wobei der Meßwert zusammen mit der Meßstellennummer kontinuierlich angezeit wird; Analogausgänge für Schreiber, wobei beliebige Meßstellen über die Eingabetastatur auf verschiedene Ausgänge geschaltet werden können.

Das Prüfprogramm umfaßt folgende Möglichkeiten: Fernprüfung der Meßleitung auf etwaigen Geberbruch und Geberkurzschluß durch z.B. äußere Gewalteinwirkung; zykli sche Prüfdurchführung; Prüfprogrammerfassung aller Meßumformerfunktionen und Grenzwertsignalverarbei tungen; gruppenwei se Zusammenfas sung der Meßumformer und Überprüfung durch unabhängige Unterrechner; Zurückhaltung etwaiger Fehlsignale oder Fehlmessungen in der Meßwertverarbeitung im Falle eines etwaigen Meßumformer- oder Rechnerausfalls.

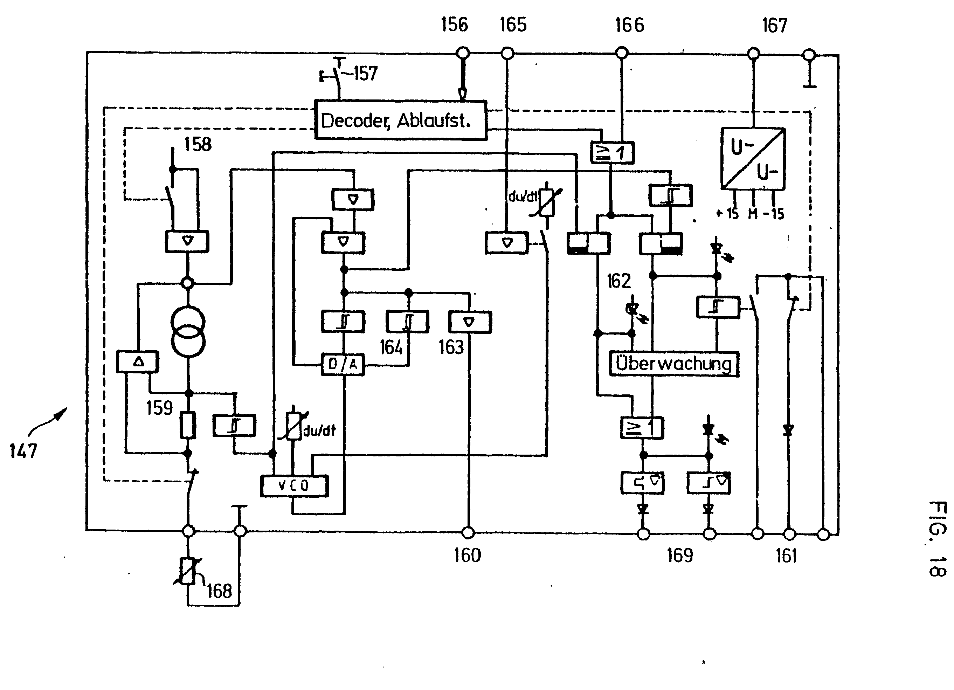

Der in der FIG. 18 dargestellte Temperaturmeßumformer l47weist folgende Funktionen auf: Verstärkerprüfung 156 sowie Prüfung der Meßwertverarbeitung und Geberbruch, Prüftaste 157 zu manuellen Prüfung, SollwertKonstantstromquelle 158, Meßbereichswiderstandl59 umschaltbar, Analogausgang 160, Grenzwertausgang 161, potentialfrei und Anzeiger auf der Frontplatte, Geberbruchanzeige 162 auf der Frontplatte, Komparatornachführung 163 ( Stop), Komparatornachführung 164 ( höhertiefer), Gradientenums chaltungseingang 165, Quittierung 166, 24 V-Eingang 167, Temperaturfühler 168 und Meßstöranzeige 169. Die technischen Daten des Temperaturmeßumformers 147 beim vorliegenden Ausführungsbeispiel umfassen einen Berei ch von 100 bis 1000 Ohm, eine maximale Meßspanne plus/minus 200 Ohm und eine maximale Arbeitbereichs-Gleichregelung plus/minus 200 Ohm. Nach Inbetriebnahme oder Beseitigung einer etwaigen Geberbruchmeldung erfolgt automatisch ein Nullabgleich des Meßumformers. Der Abgleichregler kompensisrt etwaige Widerstandsänderungen in der Leitung zum Meßfühler. Die maximal einstellbare Meßspanne 'Dynamische Änderung' umfaßt plus/minus 200 Ohm, während die minimale einstellbare Meßspanne 'Dynamische Änderung' plus/minus 5 Ohm beträgt. Zudem ist eine Umschaltung du/dt( stationärer Betrieb/ Anfahrbetrieb) vorgesehen, wobei eine Enstellbarkeit du/dt zwischen 1 V/h und 1 V/sec möglich ist.

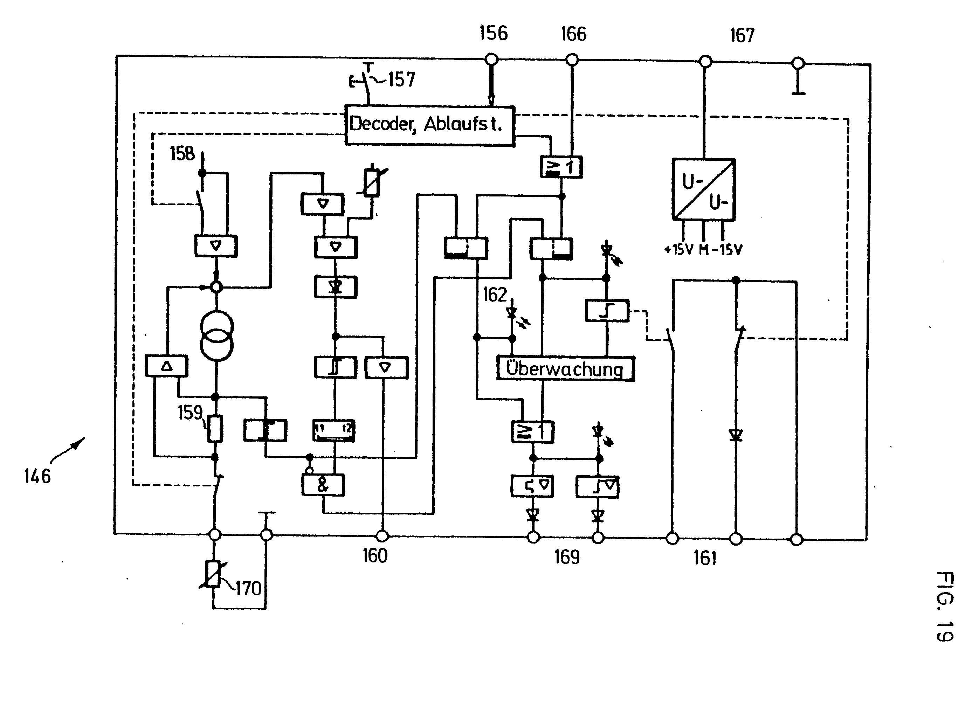

Der in der FIG. 19 dargestellte Feuchtemeßumformer 146 umfaßt ebenfalls eine Verstärkerprüfung 156, die auch der Prüfung der Meßwertverarbeitung und eines Geberbruchs dient. Auch ist eine Prüftaste 157 zur manuellen Prüfung und eine Sollwertkonstantstromquelle 158 sowie ein umschaltbarer Meßbereichswiderstand 159 vorgesehen. Außerdem sind ein Analogausgang 160, ein Grenzwertausgang 161 (potentialfrei und Anzeige auf der Frontplatte), eine Geberbruchanzeige 162 (Anzeige auf der Frontplatte), eine Quittierung 166, ein 24 V-Eingang 167 sowie eine Meßstöranzeige 169 und ein Feuchtedetektor 170 ersichtlich. Die technischen Daten des Feuchtemeßumformers 146 sind: Meßbereich 0-200 k-Ohm, 0-2 M-Ohm, 0-10 M-Ohm (0-10 V), fallende Kennlinie. GrenzwertAusgangsspannung größer als 0,5 V. Grundlastwiderstand 220 K-Ohm, 2, 2 M-Ohm, 12 M-Ohm. Die Ansprechgeschwindigkeit ist kleiner als 1 sec beim Meßbereich 10 M-Ohm, Widerstand kleiner als 9,8 M-Ohm. Die Störunterdrückung ist größer als 70 dB bei 50 Hz. Die maximale Leitungslänge beträgt ca. 3000 m.

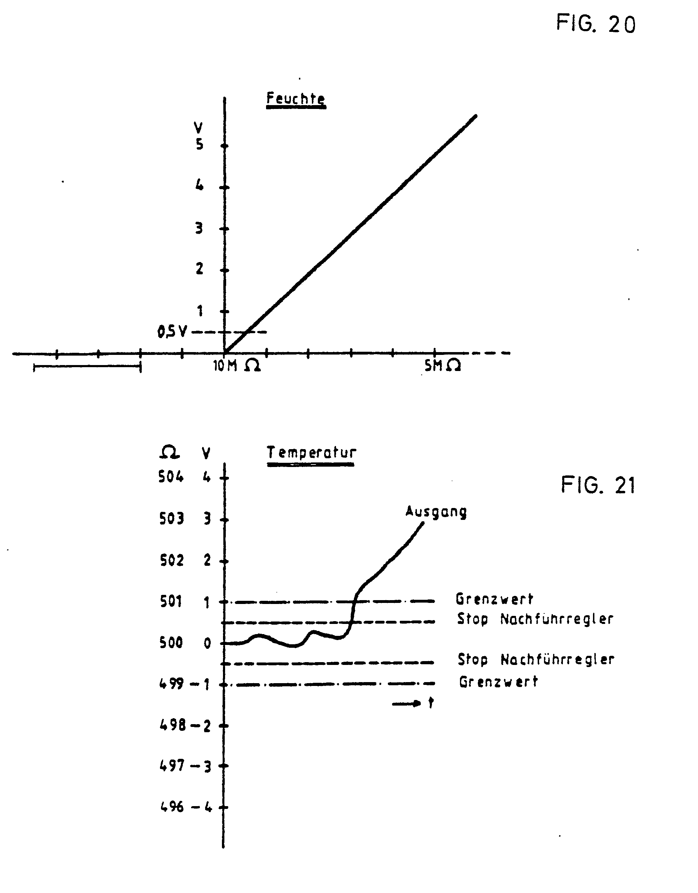

Die Figuren 20 und 21 zeigen Einstellbeispiele für den Feuchtemeßumformer 146 bzw. den Temperaturmeßumformer 147.

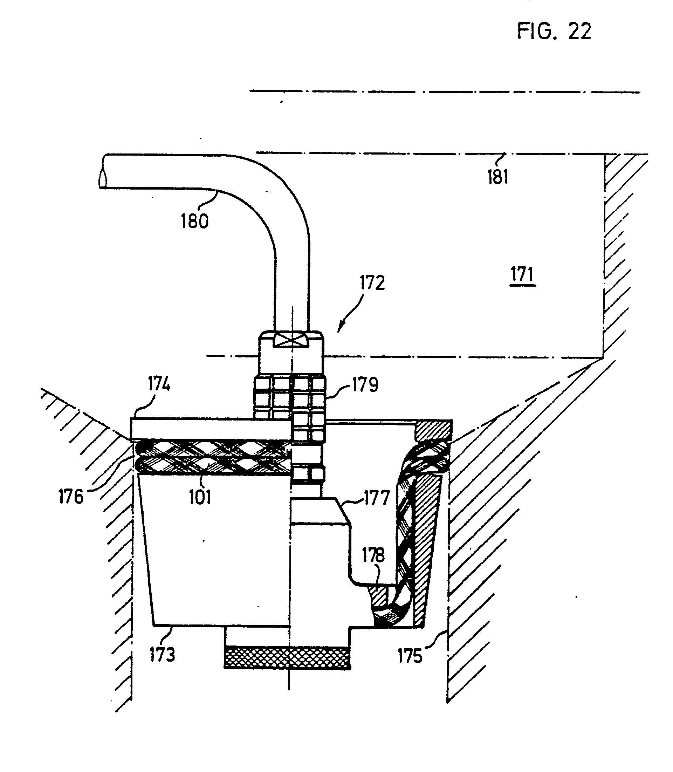

In der FIG. 22 ist in einem Gully 171, der sich im Fußboden eines Kernkraftwerkraumes befinden kann, ein Gullydetektor 172 dargestellt, der im Falle eines Einfließens von aus einem Leck austretenden Wasser im Rahmen der Überwachungseinrichtung entsprechende Signale auslöst. Hierbei ist das TemperaturFeuchte-Meßkabel 101 an einem Einsatzkörper 173 angeordnet, der sich im Bereich des Gullys 171 befindet. Der Einsatzkörper 173 besteht beim vorliegenden Ausführungsbeispiel aus einem geeigneten Kunststoffmaterial und ist nach unten hinkonisch verjüngt. Oben besitzt der Einsatzkörper 173 einen etwas größeren Umfangsrand 174, der oben auf der Behälterwandung 175 aufliegt. Unmittelbar unter dem Umfangsrand 174 ist eine Umfangsausnehmung 176 im Einsatzkörper 173 ausgebildet, in der das Temperatur-FeuchteMeßkabel 101 gelagert ist. In der Mitte des Einsatzkörpers 173 ist eine Steckerbuchse 177 feuchtigkeitsdicht verschlossen angeordnet. Die Steckerbuchse 177 wird durch radiale Stützstreben 178 gehalten. Zwischen den Stützstreben 178 befinden sich im Einsatzkörper 173 Freiräume, so daß letzterer für den Durchfluss auch eventuell größerer Mengen Wasser unten im wesentlichen offen ist. Das Sensorkabel 101 ist von der Steckerbuchse 177 durch die Stützstrebe 178 nach oben in den Bereich der Umfangsausnehmung 176 geführt, in welcher es mit zwei Umfangswindungen formschlüssig gelagert und von hier aus dann wieder durch die Stützstrebe 178 zur Steckerbuchse 177 zurückgeführt ist. Die Anordnung des Sensorkabels 101 ist so getroffen worden, daß der Durchmesser im Bereich der beiden Sensorkabelwindungen etwas größer ist als der darunter liegende Durchmesser des Einsatzkörpers 173. Zudem ist der Durchmesser im Bereich der Sensorkabelwindungen so auf den Durchmesser der Behälterwandung 175 abgestimmt, daß sich das TemperaturFeuchte-Meßkabel 101 dicht neben der Behälterwandung 175 befindet, so daß im Falle eines auch nur geringfügigen Wasserzuganges sofort eine Aufnahme vom Temperatur-Feuchte-Meßkabel 101 zwangsläufig erfolgt, wobei nicht nur ein Signal über die Feuchtigkeitsanzeige, sondern auch ein Signal über die Temperaturanzeigs erfolgt, wenn das eintreffende Leckwasser z.B. eine nur etwas andere Temperatur als die Umgebungstemperatur besitzt. In die Steckerbuchse 177 ist ein Stecker 179 einer entsprechenden (4-poligen) Meßleitung 180 flüssigkeitsdicht, aber dennoch jederzeit lösbar und wieder kuppelbar eingesetzt. Die Meßleitung 180 verläuft unter dem Gullydeckel 181 zum Randbereich des Gullys 171 und wird von hier aus zur Meßwertverarbeitung weitergeführt.

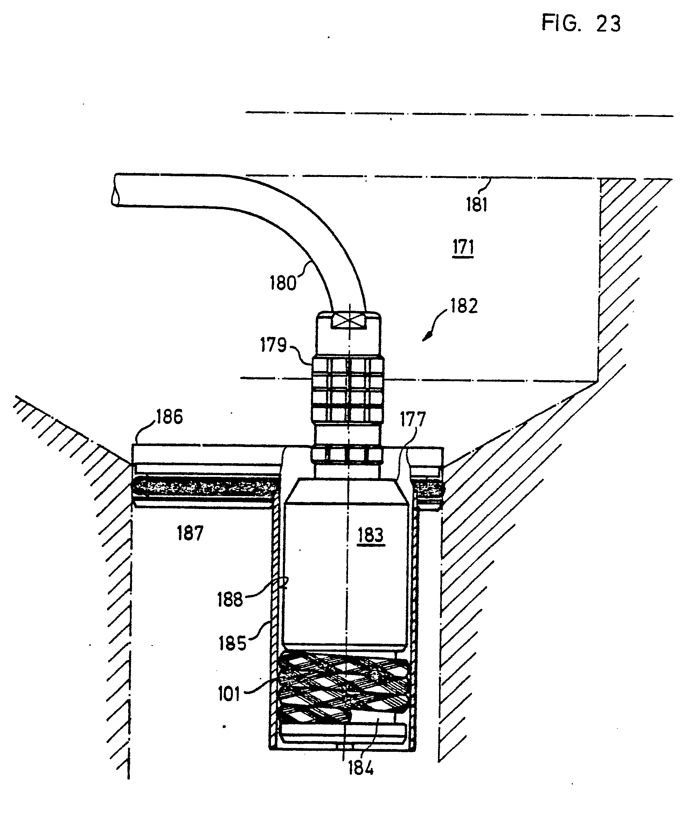

Der in der FIG. 23 dargestellte Gully-Detektor 182 ist ebenfalls in einen Gully 171 eingesetzt, der mit einem Gullydeckel 181 abgedeckt ist. Der Gullydetektor 182 umfaßt auch eine Meßleitung 180 mit einem Stecker 179, der in eine Steckerbuchse 177 eines Einsatzkörpers 183 flüssigkeitsdicht eingesteckt ist. Die Steckerbuchse 177 ist im Einsatzkörper 183 absolut dicht gelagert. Das Temperatur-Feuchte-Meßkabel 101 verläuft von der Steckerbuchse 177 im Innern des Einsatzkörpers 183 nach außen und ist hier in vorzugsweise 4 Windungen in einer Umfangsausnehmung 184 des Einsatzkörpers 183 formschlüssig gelagert und von hier aus wieder zur Steckerbuchse 177 zurückgeführt.

Der Einsatzkörper 183, der wie auch der zuvor beschriebene Einsatzkörper 173 mit der Steckerbuchse 177 und dem Temperatur-Feuchte-Meßkabel 101 als im wesentlichen einstückige und leicht zu handhabende Baueinheit ausgeführt ist, befindet sich in einem rohrförmig gestalteten Behälterteil 185, der im Bereich eines Ringes 186 außermittig an dessen Innenseitenrand befestigt ist. Der Ring 186 besitzt einen in einer Umfangsnut gelagerten 0-Ring 187, der an der Gullywandung dichtend anliegt. Im Bereich der Befestigung des Behälterteils 185 am Ring 186 ist eine muldenförmigs Flüssigkeitszuführung ausgebildet, so daß etwaiges Leckwasser an dieser Stelle zunächst zwangsgeführt in den Behälterteil 185 gelangt, wobei aufgrund der praktisch spielfreien Anordnung des Temperatur-Feuchte-Meßkabels 101 im Bereich der Behälterwandung 188 eine sofortige Signalgabe über den Feuchtemeßteil und/oder den Temperatur-Meßteil erfolgt.

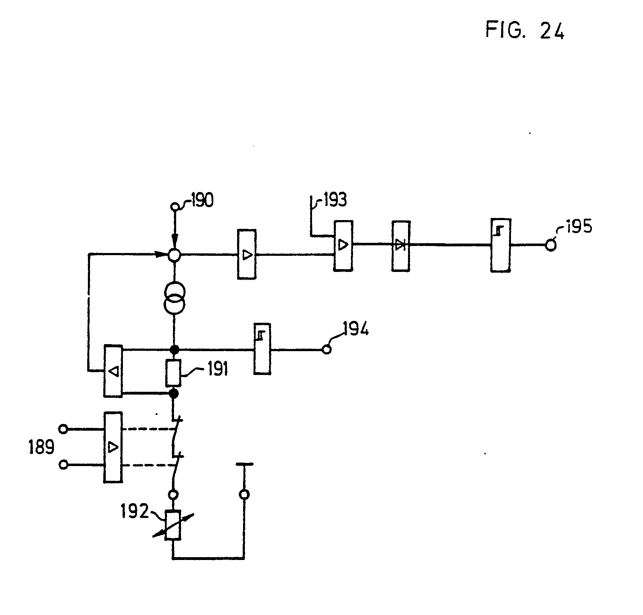

In der FIG. 24 ist ein Schema zur erfindungsgemäßen Gullyüberwachung mit folgenden Funktionen dargestellt: Prüfeingänge 189, Sollwert-Konstantstromquelle 190, Meßberei chswiderstand 191, Feuchte-/Temperaturfühler 192, Bereichsverechiebung 193 für Grundlastwiderstand, Geberbruchausgang 194 und Grenzwertausgang 195.

Die Meßwerterfaßung der Gully-Überwachungseinrichtung umfaßt somit wie auch die Dampfrohr-Überwachungseinrichtung einen Feuchtedetektor mit integriertem Temperaturfühler, wobei der als Widerstandsdraht ausgeführte Temperaturfühler bei entsprechender Spannungszuführung auch als Heizelement für die Sensorkabeltrocknung im Bereich des Gullydetektors angewendet werden kann. Das Detektionssystem ist absolut potentialfrei. Der Feuchtedetektor beinhaltet die Kupferdrähte mit variablen Grundlastwiderstand für die Empfindlichkeit und Drahtbruchüberwachung. Die Isolationen bestehen aus mehrlagig gewickeltem Glasgewebe. Es können alle in der MSR-Technik gebräuchlichen Meßkabel angeschlossen werden. Die maximale Leitungslänge beträgt ca. 3000 m. Je nach Meßfühlergrundwiderstand sind Meßbereiche zwischen 1-20 M-Ohm sinstellbar. Die Grenzwertausgangsspannung ist vorzugsweise größer als 200 mV. Die Ansprechgeschwindigkeit ist kleiner als 1 sec bei einem Meßbereich von 10 M-Ohm-0. Die Störunterdrückung ist größer als 70 dB bei 50 Hz. Die Meßwertverarbeitung erfolgt im wesentlichen vorteilhaft wie bei der zuvor beschriebenen Dampfrohrüberwachungseinrichtung und kann Meßumformer, eine Auswerteelektronik, Kontrollsysteme, Alarmgeber u. dgl. umfassen.

Das erfindungsgemäße Sensorkabel 1,101,111 kann besonders vorteilhaft im Rahmen der Erfindung auch an einem nichtisolierten Rohr, einem Behälter od. dgl. zur Erfassung etwaiger Temperatur- und/oder Feuchteäderungen angewendet werden. Dabei ist es möglich, das Temperatur-Feuchte-Meßkabel 1,101,111 zum Beispiel koaxial außen an der Rohrwandung entlang zu verlegen und mittels Halteschellen, Bändern od. dgl. zu befestigen. Auch kann das Meßkabel 1, 101, 1 1 1 zum Beispiel spiralförmig um das Rohr bzw. das zu überwachende Objekt wie Ventil, Druckausgleichsaggregat, Manometer od. dgl. gewickelt werden.

## Patentansprüche:

1. Überwachungseinrichtung für einen ein Medium beinhaltenden Behälter, insbesondere für ein Dampfrohr (2,137), zur Feststellung von Leckagen mit einem elektrischen Widerstandsdraht (6, 107) zur Temperaturkontrolle, einem feuchtigkeitsabhängigen Hygroskopisolator (5,105) und einem elektrischen Leiterdraht (3,102) zur Feuchtigkeitskontrolle sowie einem einen Temperaturmeßteil (21) und einen Feuchtemeßteil (22) aufweisenden Auswerteteil (20) für die Leckagedetektion, dadurch gekennzeichnet, daß der elektrische Widerstandsdraht (6,107) unter kontinuierlicher Abstandshaltung mittels des Hygroskopisolators (5,105) den elektrischen Leiterdraht (3,102) in Längsrichtung desselben umgibt und mit dem elektrischen Leiterdraht (3,102) sowie dem Hygroskopisolator (5,105) als baueinheitlich mehrfunktionales TemperaturFeuchte-Meßkabel (1, 101, 111) ausgebildet ist.

2. Überwachungseinrichtung nach vorstehend em Anspruch, dadurch gekennzeichnet, daß das Temperatur-FeuchteMeßkabel ( 1) zwei dem Widerstandsdraht (6) zugeordnete Anschlußpole ( 15, 15') und mindestens einen dem Leiterdraht (3) zugeordneten Polanschluß (16, 16') aufweist, die als Steckerteil (14) ausgebildet sind.

3. Überwachungseinrichtung nach einem der vorstehenden Ansprüche, dadurch gekennzeichnet, daß das TemperaturFeuchte-Meßkabel (1, 101, 111) als einen Vorführkabelteil (13, 119) und einen umgelenkten Rückführkabelteil (12, 120) aufweisende Kabels chleife (11, 118) ausgeführt ist.

4. Überwachungseinrichtung nach einem d er vorstehend en Ansprüche, dadurch gekennzeichnet, daß der Leiterdraht (102) des Temperatur-Feuchte-Meßkabels (101, 111) aus mindestens zwei im wesentlichen parallel zueinander verlauf end en Drahtleistersträngen (103, 104) gebildet ist, zwischen denen der Hygroskopisolator (105) zur Feuchtigkeitskontrolle angeordnet ist.

5. Überwachungseinrichtung nach einem der vorstehenden Ansprüche, dadurch gekennzei chnet, daß der Hygroskopisolator (5, 105) des Temperatur-Feuchte-Meßkabels (1, 101, 111) als den Leiterdraht (3) beziehungsweise die Drahtleisterstränge ( 103, 104) einzeln umgebende Hülle ausgebildet ist und aus einer Glaseidenisolation besteht.

6. Überwachungseinrichtung nach einem der vorstehenden Ansprüche, dadurch gekennzeichnet, daß der Widerstandsdraht (6, 107) des Temperatur-Feuchte-Meßkabels (1, 101, 111) auf dem Hygroskopisolator (5) beziehungsweise auf einem die Drahtleisterstränge (103, 104) umgebenden hygroskopischen Isolator, wie für sich bekannt, schraubgewind eartig angeordnet ist.

7. Überwachungseinrichtung nach einem der vorstehenden Ansprüche, dadurch gekennzeichnet, daß das Temperatur-Feuchte-Meßkabel (1, 101, 111) außen einen vorzugsweise als Glasseidengewebeschlauch ausgeführten Außenmantel (7, 108) besitzt.

8. Überwschungseinrichtung nach einem der vorstehenden Ansprüche, dadurch gekennzeichnet, daß dem Widerstandsdraht (107) für die Temperaturmessung ein Rückführleiter (109, 112) zugeordnet ist, der vorzugsweise durch Löt- oder Schweißverbindung mit dem Ende des Widerstandsdrahtes (107) verbunden und temperaturbeständig isoliert ist, wobei der Rückführleiter (109, 112) am Außenmantel (108) oder innerhalb des Außenmantels (108) parallel zu den Drahtleitersträngen (103, 104) vorgesehen ist.

9. Überwachungseinrichtung nach einem der vorstehenden Ansprüche, dadurch gekennzeichnet, daß dem Leiterdraht ( 102) des Temperatur-Feuchte-Meßkabels (101, 111) zur Feuchtedetektion und/oder Drahtbruchüberwachung ein Grundlastwiderstand (115) zwischen den beiden Drahtleitersträngen (103, 104) zugeordnet ist.

10. Überwachungseinrichtung nach einem der vorstehenden Ansprüche, gekennzeichnet durch eine frei oder festverlegte Anordnung des TemperaturFeuchte-Meßkabels (1, 101, 111) im Bereich einer eine Wandung (9, 132) des Dampfrohres (2, 137) umgebenden Wärmeisolierung (8, 130) in Längsrichtung und/oder Umfangsrichtung des Dampfrohres (2, 137), vorzugsweise nahe einer Umhüllung (10, 131) der Wärmeisolierung (8, 130).

11. Überwachungseinrichtung nach Anspruch 10, dadurch gekennzeichnet, daß das Temp eratur-FeuchteMeßkabel (1, 101, 111) in einer Nut (17, 133, 134) der Wärmeisolierung (8, 130) angeordnet und mittels eines Abdeckteils (19) abgeschirmt ist.

12. Überwachungseinrichtung nach einem der Ansprüche 10 oder 11, dadurch gekennzeichnet, daß das Temperatur-Feuchte-Meßkabel (101, 111) an einem Flachband (117) angeordnet ist, das einen im wesentlichen feinstufig nachstellbaren Spannverschluß (121) aufweist.

13. Überwachungseinrichtung nach Anspruch 12, dadurch gekennzeichnet, daß das Temperatur-FeuchteMeßkabel (101) in mindestens einer Längsnut (123) eines Spannklotzes (124) gelagert ist, der mit einem Klemmbügel (126) am Flachband (117) festgelegt ist.

14. Überwachungseinrichtung nach einem der Ansprüche 12 oder 13, dadurch gekennzeichnet, daß das Temperatur-Feuchte-Meßkabel (101) in Längsrichtung mittels mehrerer auf Abstand angeordneter Spannklötze (124) am Flachband (117) befestigt ist und daß der Vorführkabelteil ( 119) und der Rückführkabelteil ( 120) zwischen je zwei Spannklötzen (124) über Kreuz verlaufen.

15. Überwachungseinrichtung nach einem der Ansprüche 12 bis 14, dadurch gekennzeichnet, daß das Flachband (117) mit dem Temperatur-FeuchteMeßkabel (101) im Bereich einer Stoßstelle (129) der Wärmeisolierung (130) so im Bereich der Nut (131, 134) angeordnet ist, daß das Spannband (117) mittels seitlicher Begrenzungsstege (128) der eingreif enden Klemmbügel (126) und über einen gleich breiten Anschlußblock (113) in der Nut (133, 134) gegen Seitenverschiebung gesichert ist.

16. Überwachungseinrichtung nach einem der Ansprüche 1 bis 15, gekennzeichnet durch eine dem Temperatur-Feuchte-Meßkabel (1) und dem Auswerteteil (20) zugeordnete Widerstandsdrahtbruchanzeige im Temperaturmeßteil (21) und einen Leiterdrahtbruchanzeigeteil (23).

17. Überwachungseinrichtung nach einem der Ansprüche 1 bis 16, dadurch gekennzeichnet, daß mehrere Temperatur-Feuchte-Meßkabel (101) verschiedener Überwachungsstellen mit einem Unterverteiler (139) verbunden sind, daß dem TemperaturFeuchte-Meßkabel (101) eine elektronische Meßwertverarbeitung (140) mit Meßwertumformern (146, 147) zur Auswertung und Überwachung der Temperatur und Feuchte zugeordnet ist und daß der Meßwertverarbeitung (140) ein Kontrollrechnersystem (141) mit vorzugsweise zwei voneinander unabhängigen Mikrocomputern zur Datenabfrage, Störungsmeldung und Druckersteuerung zugehörig ist.

18. Überwachungseinrichtung nach einem der Ansprüche 1 bis 9, gekennzeichnet durch die Anordnung des Temperatur-Feuchte-Meßkabels (101, 111) zur Flüssigkeitsdetektion in einem Gully (171), vorzugsweise im Fußboden eines Raumes in einem Kraftwerk.

19. Überwachungseinrichtung nach Anspruch 18, dadurch gekennzeichnet, daß das Temperatur-FeuchteMeßkabel (101) an einem im Gully (171) befindlichen Einsatzkörper (173, 183) angeordnet ist, wobei zwischen einer Behälterwandung (175, 188) und dem am Einsatzkörper (173, 183) angeordneten Temperatur-FeuchteMeßkabel ( 101) ein Berührungskontakt oder ein nur geringer Spalt besteht.

20. Überwachungseinrichtung nach Anspruch 19, dadurch gekennzeichnet, daß der Einsatzkörper (173, 183) mit dem Temperatur-Feuchte-Meßkabel (101) und einer im Einsatzkörper (173, 183) feuchtigkeitsdicht isolierten Steckerbuchse (177) für einen flüssigkeitsdicht kuppelbaren Stecker (179) einer Meßleitung (180) im wesentlichen als einstückige Baueinheit ausgeführt ist.

21. Überwachungseinrichtung nach einem der Ansprüche 19 oder 20, dadurch gekennzeichnet, daß dem im Gully (171) befindlichen, am Einsatzkörper (173, 183) angeordneten TemperaturFeuchte-Meßkabel (101) eine Meßwertverarbeitung vorzugsweise mit Meßumformern, Auswerteelektronik, Kontrollsystem, Alarmgeber und dergleichen zugeordnet ist.

22. Überwachungseinrichtung nach einem der Ansprüche 1 bis 21, dadurch gekennzeichnet, daß dem Widerstandsdraht ( 107) des Temperatur-Feuchte-Meßkabels (101, 111) eine elektrische Spannung derart zuführbar ist, daß der Widerstsndsdraht (107) als elektrisches Heizelement zur Trocknung etwaiger Feuchtanteile im Temperatur-Feuchte-Meßkabel (101, 111) und/oder in dessen Umgebung ausgebildet ist.

12

**073 322**

**Claims**

1. A surveillance provision for a medium-containing vessel - more especially for pipes carrying steam (2,137) that shall detect leaks in the same, by means of the following: an electrical resistance wire (6,107) for the monitoring of temperature, a humidityindependant hygroscopic insulator (5,105) and an electrical conducting wire (3,102) for the monitoring of humidity, as well as an evaluation unit (20) for the detection of leaks comprising of a temperature measuring unit (21) and a humidity measuring unit (22), distinguished by the fact that the electrical resistance wire (6,107) is disposed spirally around the electrical conducting wire (3,102) in the longitudinal direction, being held at a constant distance from the same by the hygroscopic insulator (5,105); the electrical resistance wire together with the electrical conducting wire (3,102) and the hygroscopic insulator (5,105) form a multifunctional temperature-humidity-measuring cable (1,101,111).

2. A surveillance provision according to claim 1 wherein the temperature-humidity-measuring cable (1) is provided with at least two connection poles (15,15') on the resistance wire (6), and at least one connection pole (16,16') on the conducting wire (3),and which are constructed into a connector part (14).

3. A surveillance provision according to one of the above claims wherein the temperature-humidity measurement cable (1,101,111) consists of a feed cable (13,119), a cable loop (11,118) and a return cable (12,120).

4. A surveillance provision according to-one of the above claims wherein the conducting wire (102) of the temperature-humidity measuring cable (101,111) consists of at least two substantially parallel conducting wires (103,104), between which the hygroscopic insulator (105) for the humidity monitoring is disposed.

5. A surveillance provision according to one of the above claims wherein the hygroscopic insulator (5,105) of the temperaturehumidity measuring cable (1,101,111) forms a sheath around the conducting wire (3), that is around the two strands of the conducting wire (103,104) separately, and is in the form of a glass-silk insulation.

6. A surveillance provision according to one of the above claims wherein the resistance wire (6,107) of the temperature-humidity measuring cable (1,101,111) is wound spirally around the hygroscopic insulator (5), that is, around the hygroscopic insulator which surrounds the two wire conducting strands (103,104).

7. A surveillance provision according to one of the above claims wherein the temperature-humidity measurement cable (1,101,111) possesses an outer jacket (7,108), consisting preferably of a glass-silk fabric sleeve.

8. A surveillance provision according to one of the above claims wherein the return line (109, 112) is coordinated to the resistance wire (107) for temperature measurement, is connected to the end of the resistance wire (107), preferably by means of a solder or weld joint which is temperature-stable insulated, whereby it is intended that the return line (109, 112) should lie on the outer jacket (108) or inside the outer jacket (108) parallel to the conducting wires (103,104).

9. A surveillance provision according to one of the above claims wherein a base load resistor (115), disposed between the two wire conducting strands (103,104), is coordinated to the electrical conductor wire (102) of the temperature-humidity measurement cable (101,111) for humidity detection and/or rupture surveillance.

10. A surveillance provision according to one of the above claims wherein the temperature-humidity measurement cable (1,101,111) is disposed substantilly loose or fixed along the longitudinal axis or in a circumferential direction around the thermal insulation (8,130) on the wall (9,132) of the steam pipe (2,137), preferably near to a sheathing (10,131) of the thermal insulation (8,130).

11. A surveillance provision according to claim 10 wherein the temperature-humidity measurement cable (1,101,111) is disposed in a groove (17,133,134,) of the thermal insulation (8,130) and is protected by a covering part (19).

12. A surveillance provision according to one of the claims 10 or 11 wherein the temperature-humidity measurement cable (101,111) is attached to a flat band (117) which is provided with a toggletype fastening clamp (121) that is adjustable in fine steps.

13. A surveillance provision according to claim 12 wherein the temperature-humidity measurement cable (101) is held in position in at least one longitudinal groove (123) of a clamping block (124); this clamping block (124) is attached to the flat band (117) by means of a clamp strap (126).

14. A surveillance provision according to claims 12 or 13 wherein the temperature-humidity measurement cable (101) is attached along the flat band (117) by means of several clamping blocks (124) disposed at regular intervals, and wherein the feed cable (119) and the return cable (120) cross over between every second clamping block (124).

15. A surveillance provision according to one of the claims 12, 13 or 14 wherein the flat band (117) with the temperature-humidity measurement cable (101) is secured against a sideways shifting in the proximity of a joint (129) in the thermal insulation (130) by means of the following disposition in the groove band (117) is held in position by limiting webs (128) of the engaging clamping straps (126) and by a terminal block (113) of equal width in the groove (133,134).

16. A surveillance provision according to claims 1 to 15 wherein a resistance wire rupture indicator in the temperature measurement part (21), and a conducting wire rupture indicator (23) are both coordinated to the temperature-humidity measurement cable (1) and the data evaluation unit (20).

17. A surveillance provision according to claims 1 to 16 wherein several temperature-humidity measurement cables (101) from different surveillance positions are connected to a subsidiary distributing box (139), so that the temperature-humidity measurement cable (101) is coordinated with an electronic data processor (140) that has a transducer (146,147) for the analysis and monitoring of temperature and humidity data, and so that the data

13

processor (140) is coordinated with a monitoring computer system (141) with preferably two independant microcomputers for data collection, interference reporting and printer control.

18. A surveillance provision according to claims 1 to 9 wherein, for the detection of the presence of liquids, the temperaturehumidity measurement cable (101,111) is disposed in a gully (171), preferably in the floor of a room in a power plant.

19. A surveillance provision according to claim 18 wherein the temperature-humidity measurement cable (101) is fitted to a device (173,183) situated in the gully (171) so that there is either contact or only a slight gap between the container wall (175,188) and the temperature-humidity measurement cable (101) fitted to the device (173,183).

20. A surveillance provision according to claim 19 wherein the device (173,183) together with the temperature-humidity measurement cable (101) and a moisture-proof, insulated socket (l77), situated in the device (173,183), which is for the receival of a liquidproof, connectable plug (179) of a measurement cable (180), form substantially one construction unit.

21. A surveillance provision according to one of the claims 19 or 20, wherein the temperature-humidity measurement cable (101) which is fitted to the device (173,183) in the gully (171), is coordinated to a data processor which consists preferably of a transducer, electronics for data evaluation, monitoring system, interference alarm system and other similar devices.

22. A surveillance provision according to the claims 1 to 21 wherein the resistance wire (107) of the temperature-humidity measurement cable (101,111) must be provided with an electrical voltage such that the resistance wire (107) will function as an electrical heating element for the drying of any damp parts in the temperature-humidity measurement cable (101,111) and / or of any damp parts in the proximity of this cable.

## Revendications

1. Dispositif de contrôle servant à la détermination de fultes, pour un récipient comportant un milieu, en particulier pour un tuyau à vapeur (2,137). Ce dispositif est utilisé pour le contrôle de température à l'aide d'un fil de résistance électrique (6,107), pour le contrôle d'humidité à l'aide d'un isolateur hygroscopique dépendant de l'humidité (5,105) et à l'aide d'un fil conducteur électrique (3,102) et pour la détection de fuites à l'aide d'un élément d'identification (20) présentant un élément de mesure de température (21) et un élement de mesure d'humidité (22). Ce dispositif se caractérisé de la façon suivante: tout en maintenant l'écartement continuel au moyen de l'isolateur hygroscopique (5,105), le fil de résitance électrique (6, 107) entoure le fil conducteur électrique (3,102) dans son sens longitudinal. Il est en outre construit comme câble de mesure de température et d'humidité à plusieurs fonctions (1,101,111).

2. Dispositif de contrôle conforme à la spécification précédente et caractérisé comme suit: le câble de mesure de température et d'humidité (1) présente deux bornes de raccord (15,15') attribuées au fil de résistance (6) et un raccord polaire au moins (16,16') attribué au fil conducteur (3) et construits comme prise mâle (14).

3. Dispositif de contrôle conforme à l'une des spécifications précédentes et caractérisé comme suit: le câble de mesure de température et d'humidité (1,101,111) est construit comme une boucle de câble (11,118) presentant un élément de câble d'avance (13,119) et un élément de câble de retour, renversé (12,120).

4. Dispositif de contrôle conforme à l'une des spécifications précédentes et caractérisé comme suit: le fil conducteur (102) du câble de mesure de température et d'humidité (101,111) est constitué d'au moins deux faisceaux de fil conducteur (103,104) paralleles l'un à l'autre et entre lesquels est disposé l'isolateur hygroscopique (105) destiné au contrôle de l'humidité.

5. Dispositif de contrôle conforme à l'une des spécifications précédentes et caractérisé comme suit: l'isolateur hygroscopique (5,105) du câble de mesure de température et d'humidité (1,101,111) est conçu comme une gaine enveloppant séparemment le fil conducteur (3) respectivement les faisceaux du fil conducteur (103,104) et constitué d'une isolation en soie de verre.

6. Dispositif de contrôle conforme à l'une des spécifications précédentes et caractérisé comme suit: le fil de résistance (6, 107) du câble de mesure de température et d'humidité (1,101,111) est placé, semblable à un filet de vis, sur l'isolateur hygroscopique (5), respectivement sur un isolateur hygroscopique enveloppant les faisceaux du fil conducteur (103,104).

7. Dispositif de contrôle conforme à l'une des spécifications précédentes et caractérisé comme suit: le càble de mesure de température et d'humidité (1,101,111) est muni à l'extérieur d'une gaine (7, 108), choisie de préférence en soie de verre.

8. Dispositif de contrôle ccnforme à l'une des spécifications précédentes et caractérisé comme suit: le fil de résistance (107), destiné à la mesure de la température est muni d'un conducteur de retour (109,112), lie à l'extrémité du fil de résistance (107) par brasage ou soudage de préférence et isolé contre les variations de température; le conducteur de retour (109,112) est posé sur la gaine extérieure (108) ou à l'intérieur de la gaine extérieure (108), parallèlement aux faisceaux de fil conducteur (103, 104).

9. Dispositif de contrôle conforme à l'une des spécifications précédentes et caractérisé comme suit: le fil conducteur (102) du câble de mesure de température et d'humidité (101,111) est muni, entre les deux faisceaux de fils condcteurs (103, 104) d'une résistance de charge de base (115), servant à détecter de l'humidité et/ ou pour surveiller une rupture éventuelle du fil.

10. Dispositif de contrôle conforme à l'une des spécifications précédentes et caractérisé par une disposition

14

libre ou fixé du câble de mesure de température et d'humidité (1,101,111) en sens longitudinal et à proximité d'un isolant thermique (8,130) enveloppant une paroi (9, 132) du tuyau de vapeur (2, 137) et/ou à l'entour du tuyau de vapeur, de préférence à proximité de la gaine (10,131) de l'isolant thermique (8,130).

11. Dispositif de contrôle conforme à la spécification 10 et caractérisé comme suit: le câble de mesure de température et d'humidité (1,101,111) est posé dans une rainure (17,133,134) de l'isolant thermique (8,130) et protégé au moyen d'une pièce de protection (19).

12. Dispositif de contrôle conforme aux spécifications 10 ou 11 et caractérisé comme suit: le câble de mesure de température et d'humidité (101,111) est installé sur une bande plate (117), munie d'une fermeture à genouillère (121) à régulation sensible.

13. Dispositif de contrôle conforme à la spécification 12 et caractérisé comme suit: le câble de mesure de température et d'humidité (101) est logé dans au moins une rainure longitudinale (123) d'une cale de serrage (124), fixée à la bande plate (117) au moyen d'un étrier de serrage (126).

14. Dispositif de contrôle conforme à l'une des spéoifications 12 ou 13 et caractérisé comme suit: le câble de mesure de température et d'humidité (101) est fixé à la bande plate (117) dans le sens longitudinal, au moyen de plusieurs cales de serrage (124) placées à distances. Autre caractéristique: le câble d'avance (119) et le câble de retour (120) passent, en croix, entre chacune des deux cales de serrage (124).

15. Dispositif de contrôle conforme à l'une des spécifications 12 à 14 et caractérisé comme suit: la bande plate (117) et le câble de mesure de température et d'humidité (101) se trouvant à proximité d'un joint (129) de l'isolant thermique (130) est placée dans le rayon de la rainure (131,134) de telle façon que le ruban de tenslon (117) soit protégé dans la rainure (133,134) contre un déplacement latéral et ce, au moyen des barrettes de limitation latérales (128) des étriers de serrage (126) et par l'intermédiaire d'un bloc de connexion (113) de largeur égale.

16. Dispositif de contrôle conforme à l'une des spécifications 1 à 15 et caractérisé par une signalisation de rupture du fil de résistance dans l'élément de mesure de température (21), attribuée au càble de mesure de température et d'humidité (1) et à l'élément d'évaluation (20) et caractérisé également par un élément de signalisation de rupture du fil conducteur (23).

17. Dispositif de contrôle conforme à l'une des spécifications 1 à 16 et caractérisé comme suit: plusieurs câbles de mesure de température et d'humidité (101) de différents points de contrôle sont reliés à un distributeur secondaire (139). Un traitement électronique des données (140) avec convertisseurs (146,147) pour l'évaluation et le contrôle de la température et de l'humidité est attribué au câble de mesure de température et d'humidité (101). Un calculateur de contrôle (141) avec deux micro-ordinateurs, de préférence indépendants l'un de l'autre et destinés à l'appel des données, à la signalisation des perturbations et à la commande de l'imprimante fait partie du traitement des données de mesure (140).

18. Dispositif de contrôle conforme à l'une des spécifications 1 à 9 et caractérisé par l'installation du câble de mesure de température et d'humidité (101,111) dans une bouche d'égout (171), de préférence dans le sol d'un local d'une usine électrique, pour la détection de liquides.

19. Dispositif de contrôle conforme à la spécification 18 et caractérisé comme suit: le câble de mesure de température et d'humidité (101) est installé à une pièce d'insertion (173, 183) se trouvant dans la bouche d'égout (171); toutefois, entre une paroi du récipient (175, 188) et le câble de mesure de température et d'humidité (101) installé à la pièce d'insertion (173,183) ne doit exister qu'un point de contact ou un interstice minime.

20. Dispositif de contrôle conforme à la spécification 19 et caractérisé comme suit: le corps d'insertion (173, 183) avec le câble de mesure de température et d'humidité (101) et une fiche femelle (177), isolée contre l'humidité, se trouvant dans le corps d'insertion (173,183) et destinée à une fiche mâle étanche et pouvant être accouplée (179), d'un circuit de mesure (180) est construit essentiellement comme unité modulaire d'une seule pièce.

21. Dispositif de contrôle conforme à l'une des spécifications 19 ou 20 et caractérisé comme sult: un traitement des valeurs de mesure avec convertisseurs de mesure, électronique d'interprétation, système de contrôle, système d'alarme et autres est attribué au càble de mesure de température et d'humidité (101) se trouvant dans la bouche d'égout (171) et installé au corps d'insertion (173,183).

22. Dispositif de contrôle conforme à l'une des spécifications 1 à 21 et caractérisé comme suit: il est possible d'alimenter en tension électrique le fil de résistance (107) du câble de mesure de température et d'humidité (101,111) de telle façon qu'il puisse fonctionner comme élément de chauffage électrique pour le séchage de pièces humides se trouvant dans le câble de mesure de température et d'humidité (101,111) et/ou à proximité de ce câble de mesure.

FIG. 1

FIG. 2

8  9  2  14

11  13  12  1  10  16  16'  15  15'

FIG. 3

19  17  18  10

1  9  8

2

FIG. 4

1  3  5  6

24  25

23  22  21  20

3

FIG. 5

FIG. 6

FIG. 7

115

101

113

114

FIG. 10

FIG. 9

FIG. 8

FIG. 11

FIG. 12

FIG. 13

FIG. 14

FIG. 15

FIG.16

FIG. 17

FIG. 18

19

073 322

FIG. 19

21

**FIG. 20**

Feuchte

**FIG. 21**

Temperatur

Ausgang

Grenzwert
Stop Nachführregler
Stop Nachführregler
Grenzwert

FIG. 22

FIG. 23

181

171

182

180

179

186

177

187

188

185

101

183

184

FIG. 24